(19) Europäisches Patentamt / European Patent Office / Office européen des brevets

(11) **EP 4 559 504 A2**

(12) **EUROPEAN PATENT APPLICATION**

(43) Date of publication:
28.05.2025 Bulletin 2025/22

(21) Application number: 25170633.9

(22) Date of filing: 05.04.2021

(51) International Patent Classification (IPC):
*A61M 25/00* *(2006.01)*

(52) Cooperative Patent Classification (CPC):
**A61F 2/2436;** A61F 2/2418; A61F 2250/0018;
A61F 2250/0039; A61M 25/0067; A61M 25/0074

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: 06.04.2020 US 202063005905 P
10.09.2020 US 202063076783 P
06.11.2020 US 202063110846 P

(62) Document number(s) of the earlier application(s) in
accordance with Art. 76 EPC:
**21722016.9 / 4 132 425**

(71) Applicant: **Edwards Lifesciences Corporation
Irvine, CA 92614 (US)**

(72) Inventors:
• **Cummings, Brendan Christopher
Irvine, CA 92614 (US)**
• **Manzella, Salvatore Jr.
Irvine, CA 92614 (US)**

(74) Representative: **Eisenführ Speiser
Patentanwälte Rechtsanwälte PartGmbB
Gollierstraße 4
80339 München (DE)**

Remarks:
This application was filed on 15.04.2025 as a
divisional application to the application mentioned
under INID code 62.

(54) **PROSTHETIC HEART VALVE DELIVERY APPARATUS**

(57) Disclosed herein is a delivery apparatus used to introduce a prosthetic device, such as a heart valve or other implant, into a patient's vasculature. The delivery apparatus includes a sheath member defining a tubular structure and a tip portion provided at the distal end of the sheath member. The inner surface of the tip portion includes structure for directing expansion of the heart valve during delivery and/or also directing folding and/or crimping of the prosthetic heart valve as it is withdrawn back into the sheath.

FIG. 28

**EP 4 559 504 A2**

## Description

### CROSS REFERENCE TO RELATED APPLICATIONS

This application claims priority to U.S. Provisional Application No. 63/005,905 (Attorney Docket No. 10197US01), filed April 6, 2020, U.S. Provisional Application No. 63/076,783 (Attorney Docket No. 10197US02), filed September 10, 2020, and U.S. Provisional Application No. 63/110,846 (Attorney Docket No. 10197US03), filed November 6, 2020, the disclosures of which are incorporated by reference in their entirety.

### FIELD

The present disclosure concerns implementations of a delivery apparatus for use with catheter-based technologies to introduce a prosthetic device, such as a heart valve or other implant, into the patient's vasculature.

### BACKGROUND

Prosthetic cardiac valves have been used for many years to treat cardiac valvular disorders. The native heart valves (such as the aortic, pulmonary and mitral valves) serve critical functions in assuring the forward flow of an adequate supply of blood through the cardiovascular system. These heart valves can be rendered less effective by congenital, inflammatory or infectious conditions. Such damage to the valves can result in serious cardiovascular compromise or death. For many years the definitive treatment for such disorders was the surgical repair or replacement of the valve during open heart surgery, but such surgeries are prone to many complications. More recently a transvascular technique has been developed for introducing and implanting a prosthetic heart valve using a flexible catheter in a manner that is less invasive than open heart surgery.

In this technique, an introducer sheath can be used to safely introduce a delivery apparatus into a patient's vasculature (e.g., the femoral artery). An introducer sheath generally has an elongated sleeve that is inserted into the vasculature and a housing that contains one or more sealing valves that allow a delivery apparatus to be placed in fluid communication with the vasculature with minimal blood loss.

A prosthetic valve is then mounted in a crimped state on the end portion of a flexible catheter and advanced through a blood vessel of the patient via the introducer sheath until the prosthetic valve reaches the implantation site. The prosthetic valve at the catheter tip is then expanded to its functional size at the site of the defective native valve such as by inflating a balloon on which the prosthetic valve is mounted. Alternatively, the prosthetic valve can have a resilient, self-expanding stent or frame that expands the prosthetic valve to its functional size when it is advanced from a delivery sheath at the distal end of the catheter.

Balloon-expandable prosthetic valves can be used when replacing calcified native valves. Advantageously, the catheter balloon can apply sufficient expanding force to anchor the frame of the prosthetic valve to the surrounding calcified tissue. Self-expanding prosthetic valves can be used to replace a defective, non-stenotic (non-calcified) native valve, although they also can be used to replace stenotic valves. One drawback associated with implanting a self-expanding prosthetic valve is that as the operator begins to advance the prosthetic valve from the open end of the delivery sheath, the prosthetic valve tends to "jump" out very quickly from the end of the sheath; in other words, the outward biasing force of the prosthetic valve's frame tends to cause the prosthetic valve to be ejected very quickly from the distal end of the delivery sheath, making it difficult to deliver the prosthetic valve from the sheath in a precise and controlled manner and increasing the risk of trauma to the patient. Additionally, during a procedure it may be necessary to reposition and/or withdraw the prosthetic valve if the valve is deployed in the wrong location or appears incompatible with the patient anatomy. However, there are several problems with (fully or partially) re-sheathing a self-expanding prosthetic valve. For example, as the implant exhibits larger outward radial forces the smaller is constrained, making it more difficult to deploy and retract. The implant may also compress in unpredictable and non-uniform ways, causing it to get stuck in the sheath and potentially become unusable/incapable of being re-deployed. Thus, there remains a need for further improvements in delivery catheters used in endovascular systems used for implanting and repositioning heart valves and other prosthetic devices.

### SUMMARY

Certain examples in the present disclosure provide a prosthetic valve (e.g., a prosthetic heart valve) and a valve delivery apparatus for delivery of the prosthetic valve to a native valve site via the human vasculature. The delivery apparatus is particularly suited for advancing a prosthetic valve through the aorta (i.e., in a retrograde approach) for replacing a diseased native aortic valve. The delivery apparatus in particular implementations is configured to deploy a prosthetic valve from a delivery sheath in a precise and controlled manner at the target location within the body.

Another example delivery sheath system disclosed herein comprises a sheath member defining a tubular structure extending between a proximal end and a distal end of the sheath member. A tip portion is provided at the distal end of the sheath member, the tip portion having a central lumen extending therethrough. At least one cross-sectional segment of an inner surface of the tip portion includes alternating portions of concave and convex curvature.

Also disclosed herein is a method of deploying a prosthetic heart valve that comprises inserting an introducer sheath into a blood vessel and inserting a delivery sheath into a central lumen of the introducer sheath, with a prosthetic valve disposed therein. The delivery sheath comprising a sheath member defining a tubular structure extending between a proximal end and a distal end of the sheath member, and a tip portion provided at the distal end of the sheath member, the tip portion having a central lumen extending therethrough, wherein at least one cross-sectional segment of an inner surface of the tip portion includes alternating portions of concave and convex curvature. The prosthetic valve is advanced through the tip portion to a treatment site such that the prosthetic valve at least partially expands within the tip portion. The alternating portions of concave and convex curvature facilitate directed expansion of the prosthetic heart valve as it passes through the tip portion.

Another example delivery sheath system disclosed herein comprises a sheath member defining a tubular structure extending between a proximal end and a distal end of the sheath member, a ring portion provided at the distal end of the sheath member, and a tip portion extending from a distal end of the ring portion. The tip portion includes a longitudinally extending folding region movable between a folded and unfolded configuration. In the folded configuration, the tip portion creases along a longitudinally extending edge such that circumferential portions of the tip portion at least partially overlap. In the unfolded configuration, the tip portion defines an increasing tapered surface having a larger diameter at a distal end of the tip portion.

Further disclosed herein is a method of deploying a prosthetic valve that comprises inserting an introducer sheath into a blood vessel and inserting a delivery sheath into a central lumen of the introducer sheath, with a prosthetic valve disposed therein. The delivery sheath comprises a sheath member that defines a tubular structure extending between a proximal end and a distal end of the sheath member, a ring portion provided at the distal end of the sheath member, and a tip portion extending from a distal end of the ring portion. The tip portion includes a longitudinally extending folding region movable between a folded and unfolded configuration. In the folded configuration, the tip portion creases along a longitudinally extending edge such that circumferential portions of the tip portion at least partially overlap. In the unfolded configuration, the tip portions defines an increasing tapered surface having a larger diameter at a distal end of the tip portion. The prosthetic valve is advanced through the distal end of the sheath member, to the ring portion, through the ring portion to the tip portion, through the tip portion such that the prosthetic valve at least partially expands within the tip portion and the tip portion moves from the folded to the unfolded configuration in response to the outward directed radial force of the prosthetic heart valve passing through the tip portion. The prosthetic valve is then advanced beyond a distal end of the delivery sheath to a treatment site.

Another example delivery sheath disclosed herein comprises: a sheath member defining a tubular structure extending between a proximal end and a distal end of the sheath member and a tip portion provided at the distal end of the sheath member. The tip portion has a central lumen extending therethrough. The tip portion is discontinuous and has two flaps extending circumferentially around the tip portion. The tip portion is movable between an unexpanded and an expanded configuration. In the unexpanded configuration, the tip portion defines a constant diameter along its length. In the expanded configuration, the tip portion flares open increasing a diameter of the distal end of the tip portion and increasing the spacing between the two flaps.

An example method of deploying a prosthetic valve disclosed herein comprises inserting an introducer sheath into a blood vessel, inserting a delivery sheath into a central lumen of the introducer sheath, with a prosthetic valve disposed therein. The delivery sheath comprises a sheath member defining a tubular structure extending between a proximal end and a distal end of the sheath member, a tip portion provided at the distal end of the sheath member, the tip portion having a central lumen extending therethrough. The tip portion is discontinuous and has two flaps extending circumferentially around the tip portion. The tip portion is movable between an expanded and an unexpanded configuration. In the unexpanded configuration, the tip portion defines a constant diameter along its length. In the expanded configuration, the tip portion flares open increasing a diameter of the distal end of the tip portion and increasing the spacing between the two flaps. The prosthetic valve is advanced through the distal end of the sheath member, to the tip portion, through the tip portion to a treatment site such that the prosthetic valve at least partially expands within the tip portion and the tip portion moved from the unexpanded to the expanded configuration in response to the outward directed radial force of the prosthetic heart valve passing through the tip portion.

A further example method of deploying a prosthetic valve disclosed herein comprises inserting an introducer sheath into a blood vessel, inserting a delivery sheath into a central lumen of the introducer sheath, with a prosthetic valve disposed therein. The delivery sheath comprises a sheath member defining a tubular structure extending between a proximal end and a distal end of the sheath member and a tip portion provided at the distal end of the sheath member. The tip portion has a central lumen extending therethrough. The tip portion is discontinuous and has two flaps extending circumferentially around the tip portion. The tip portion is movable between an expanded and an unexpanded configuration. In the unexpanded configuration, the tip portion defines a constant diameter along its length. In the expanded configuration, the tip portion flares open increasing a diameter of the distal end of the tip portion and increasing the spacing between the two flaps. The prosthetic valve is advanced through the distal end of the sheath member, to the tip portion, through the tip portion beyond a distal end of the delivery sheath to a treatment site such that the prosthetic valve at least partially expands within the tip portion and the tip portion moved from the unexpanded to the expanded configuration in response to the outward directed radial force of the prosthetic heart valve passing through the tip portion.

## DESCRIPTION OF DRAWINGS

FIG. 1 is a perspective view of a prosthetic valve that can be used to replace the native aortic valve of the heart, according to one implementation.

FIG. 2 is a perspective view of a portion of the prosthetic valve of FIG. 1 illustrating the connection of two leaflets to the support frame of the prosthetic valve.

FIG. 3 is side elevation view of the support frame of the prosthetic valve of FIG. 1.

FIG. 4 is a perspective view of the support frame of the prosthetic valve of FIG. 1.

FIG. 5A is a cross-sectional view of the heart showing the prosthetic valve of FIG. 1 implanted within the aortic annulus.

FIG. 5B is an enlarged view of FIG. 5A illustrating the prosthetic valve implanted within the aortic annulus, shown with the leaflet structure of the prosthetic valve removed for clarity.

FIG. 6 is a perspective view of the leaflet structure of the prosthetic valve of FIG. 1 shown prior to being secured to the support frame.

FIG. 7 is a cross-sectional view of the prosthetic valve of FIG. 1.

FIG. 8 is a cross-sectional view of an implementation of a delivery apparatus that can be used to deliver and implant a prosthetic valve, such as the prosthetic valve shown in FIG. 1.

FIG. 9 is an exploded view of the delivery apparatus of FIG. 8.

FIG. 10 is a side view of the guide catheter of the delivery apparatus of FIG. 8.

FIG. 11A is a perspective, exploded view of the proximal end portion of the guide catheter of FIG. 10.

FIG. 11B is a perspective, exploded view of the distal end portion of the guide catheter of FIG. 10.

FIG. 12 is an enlarged side view of the distal end portion of the delivery apparatus of FIG. 8 showing the delivery sheath in a delivery position covering a prosthetic valve in a compressed state for delivery into a patient.

FIG. 13 is an enlarged cross-sectional view of a section of the distal end portion of the delivery apparatus of FIG. 8 showing the valve-retaining mechanism securing the stent of a prosthetic valve to the delivery apparatus.

FIG. 14 is an enlarged cross-sectional view similar to FIG. 13, showing the inner fork of the valve-retaining mechanism in a release position for releasing the prosthetic valve from the delivery apparatus.

FIG. 15 is a perspective view of a delivery sheath including a tip portion.

FIG. 16 is a perspective view of the delivery sheath and tip portion of FIG. 15.

FIG. 17 is a side view of the delivery sheath and tip portion of FIG. 15.

FIG. 18 is a cross-section view of the delivery sheath and tip portion.

FIG. 19 is a cross-section view of the tip portion.

FIG. 20 is a proximal end view of the tip portion.

FIG. 21 is a distal end view of the tip portion.

FIG. 22 is an enlarged side view of the distal end portion of the nose cone catheter of the delivery apparatus of FIG. 8.

FIG. 23 is an enlarged, cross-sectional view of the nose cone of the catheter shown FIG. 22.

FIG. 24 is an enlarged cross-sectional view of the distal end portion of the delivery apparatus of FIG. 8 showing the stent of a prosthetic valve retained in a compressed state within a delivery sheath.

FIG. 25 is a perspective view of a prosthetic valve partially retained within the tip portion of the delivery sheath.

FIG. 26 is a schematic side view of an example delivery sheath, tip portion and prosthetic valve.

FIG. 27 is schematic end view of a partially compressed prosthetic valve.

FIG. 28 is a perspective view of a delivery sheath including a tip portion in a folded configuration.

FIG. 29 is a perspective view of the delivery sheath and tip portion of FIG. 28 in an unfolded or expanded configuration.

FIG. 30 is an enlarged perspective view of the distal end of the delivery sheath of FIG. 28.

FIG. 31 is a perspective view of a delivery sheath including a tip portion in an unexpanded configuration.

FIG. 32 is a perspective view of the delivery sheath and tip portion of FIG. 31 in an expanded configuration.

## DETAILED DESCRIPTION

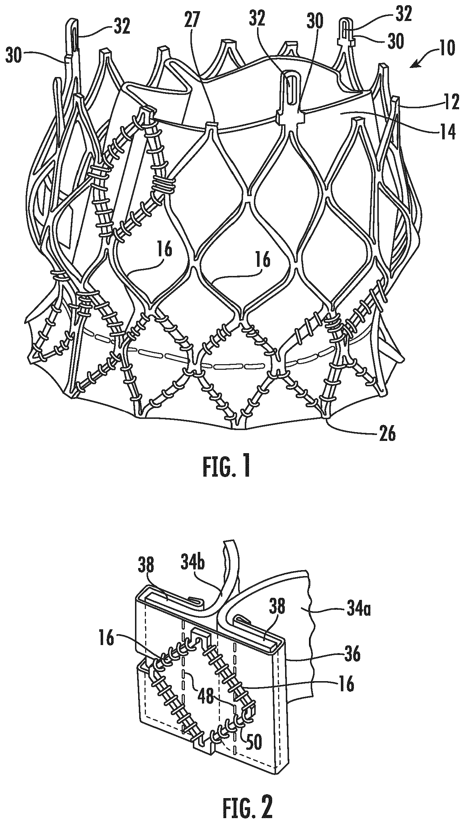

Referring first to FIG. 1, there is shown a prosthetic aortic heart valve 10, according to one implementation. The prosthetic valve 10 includes an expandable frame member, or stent, 12 that supports a flexible leaflet section 14. The prosthetic valve 10 is radially compressible to a compressed state for delivery through the body to a deployment site and expandable to its functional size shown in FIG. 1 at the deployment site. In certain implementations, the prosthetic valve 10 is self-expanding; that is, the prosthetic valve can radially expand to its functional size when advanced from the distal end of a delivery sheath. Apparatuses particularly suited for percutaneous delivery and implantation of a self-expanding prosthetic valve are described in detail below. In other implementations, the prosthetic valve can be a balloon-expandable prosthetic valve that can be adapted to be mounted in a compressed state on the balloon of a delivery catheter. The prosthetic valve can be expanded to its functional size at a deployment site by inflating the balloon, as known in the art. Example valves are described, for example, in U.S. Patent No. 9,867,700, the disclosure of which is herein incorporated by referenced in it's entirety.

The illustrated prosthetic valve 10 is adapted to be deployed in the native aortic annulus, although it also can be used to replace the other native valves of the heart. Moreover, the prosthetic valve 10 can be adapted to replace other valves within the body, such venous valves.

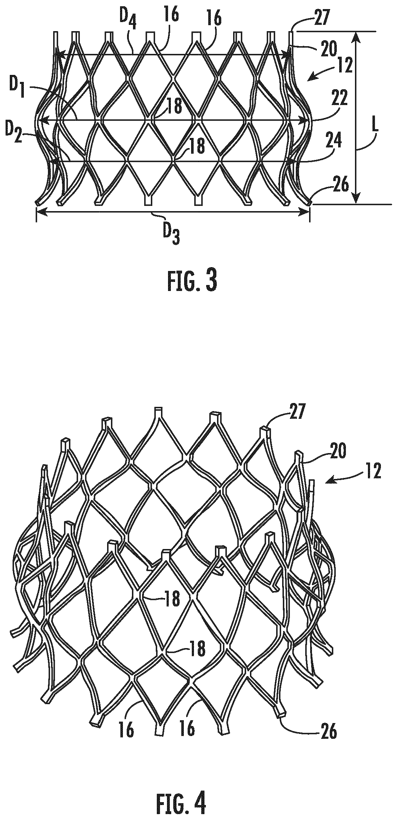

FIGS. 3 and 4 show the stent 12 without the leaflet section 14 for purposes of illustration. As shown, the stent 12 can be formed from a plurality of longitudinally extending, generally sinusoidal shaped frame members, or struts, 16. The struts 16 are formed with alternating bends and are welded or otherwise secured to each other at nodes 18 formed from the vertices of adjacent bends so as to form a mesh structure. The struts 16 can be made of a suitable shape memory material, such as the nickel titanium alloy known as Nitinol, that allows the prosthetic valve to be compressed to a reduced diameter for delivery in a delivery apparatus (such as described below) and then causes the prosthetic valve to expand to its functional size inside the patient's body when deployed from the delivery apparatus. If the prosthetic valve is a balloon-expandable prosthetic valve that is adapted to be crimped onto an inflatable balloon of a delivery apparatus and expanded to its functional size by inflation of the balloon, the stent 12 can be made of a suitable ductile material, such as stainless steel.

The stent 12 has an inflow end 26 and an outflow end 27. The mesh structure formed by struts 16 comprises a generally cylindrical "upper" or outflow end portion 20, an outwardly bowed or distended intermediate section 22, and an inwardly bowed "lower" or inflow end portion 24. The intermediate section 22 desirably is sized and shaped to extend into the Valsalva sinuses in the root of the aorta to assist in anchoring the prosthetic valve in place once implanted. As shown, the mesh structure desirably has a curved shape along its entire length that gradually increases in diameter from the outflow end portion 20 to the intermediate section 22, then gradually decreases in diameter from the intermediate section 22 to a location on the inflow end portion 24, and then gradually increases in diameter to form a flared portion terminating at the inflow end 26.

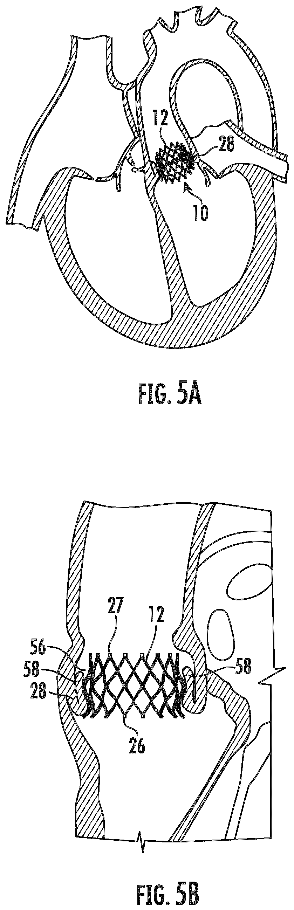

When the prosthetic valve is in its expanded state, the intermediate section 22 has a diameter $D_1$, the inflow end portion 24 has a minimum diameter $D_2$, the inflow end 26 has a diameter $D_3$, and the outflow end portion 20 has a diameter $D_4$, where $D_2$ is less than $D_1$ and $D_3$, and $D_4$ is less than $D_2$. In addition, $D_1$ and $D_3$ desirably are greater than the diameter of the native annulus in which the prosthetic valve is to be implanted. In this manner, the overall shape of the stent 12 assists in retaining the prosthetic valve at the implantation site. More specifically, and referring to FIGS. 5A and 5B, the prosthetic valve 10 can be implanted within a native valve (the aortic valve in the illustrated example) such that the lower section 24 is positioned within the aortic annulus 28, the intermediate section 24 extends above the aortic annulus into the Valsalva's sinuses 56, and the lower flared end 26 extends below the aortic annulus. The prosthetic valve 10 is retained within the native valve by the radial outward force of the lower section 24 against the surrounding tissue of the aortic annulus 28 as well as the geometry of the stent. Specifically, the intermediate section 24 and the flared lower end 26 extend radially outwardly beyond the aortic annulus 28 to better resist against axial dislodgement of the prosthetic valve in the upstream and downstream directions (toward and away from the aorta). Depending on the condition of the native leaflets 58, the prosthetic valve typically is deployed within the native annulus 28 with the native leaflets 58 folded upwardly and compressed between the outer surface of the stent 12 and the walls of the Valsalva sinuses, as depicted in FIG. 5B. In some cases, it may be desirable to excise the leaflets 58 prior to implanting the prosthetic valve 10.

Known prosthetic valves having a self-expanding frame typically have additional anchoring devices or frame portions that extend into and become fixed to non-diseased areas of the vasculature. Because the shape of the stent 12 assists in retaining the prosthetic valve, additional anchoring devices are not required and the overall length L of the stent can be minimized to prevent the stent upper portion 20 from extending into the non-diseased area of the aorta, or to at least minimize the extent to which the upper portion 20 extends into the non-diseased area of the aorta. Avoiding the non-diseased area of the patient's vasculature helps avoid complications if future intervention is required. For example, the prosthetic valve can be more easily removed from the patient because the stent is primarily anchored to the diseased part of the native valve. Furthermore, a shorter prosthetic valve is more easily navigated around the aortic arch.

In particular implementations, for a prosthetic valve intended for use in a 22-mm to 24-mm annulus, the diameter $D_1$ is about 28 mm to about 32 mm, with 30 mm being a specific example; the diameter $D_2$ is about 24 mm to about 28 mm, with 26 mm being a specific example; the diameter $D_3$ is about 28 mm to about 32 mm, with 30 mm being a specific example; and the diameter $D_4$ is about 24 mm to about 28 mm, with 26 mm being a specific example. The length L in particular implementations is about 20 mm to about 24 mm, with 22 mm being a specific example.

Referring to FIG. 1, the stent 12 can have a plurality of angularly spaced retaining arms, or projections, in the form of posts 30 (three in the illustrated implementation) that extend from the stent upper portion 20. Each retaining arm 30 has a respective aperture 32 that is sized to receive prongs of a valve-retaining mechanism that can be used to form a releasable connection between the prosthetic valve and a delivery apparatus (described below). In alternative implementations, the retaining arms 30 need not be provided if a valve-retaining mechanism is not used.

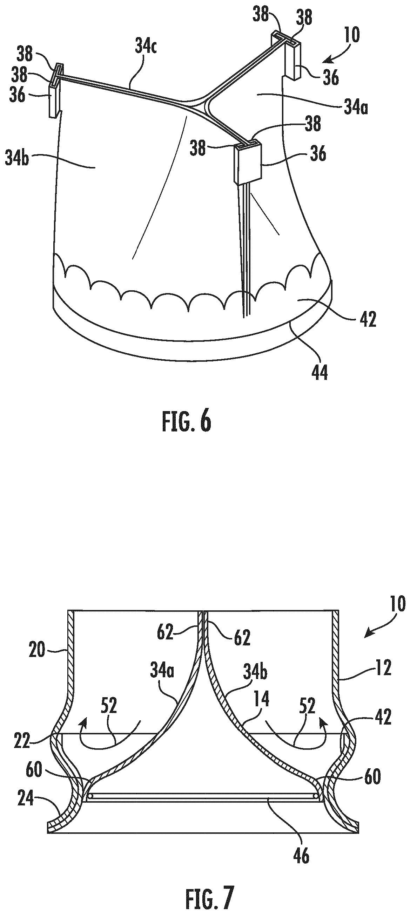

As best shown in FIGS. 6 and 7, the leaflet assembly 14 in the illustrated implementation comprises three leaflets 34a, 34b, 34c made of a flexible material. Each leaflet has an inflow end portion 60 and an outflow end portion 62. The leaflets can comprise any suitable biological material (e.g., pericardial tissue, such as bovine or equine pericadium), bio-compatible synthetic materials, or other such materials, such as those described in U.S. Patent No. 6,730,118, which is incorporated herein by reference. The leaflet assembly 14 can include an annular reinforcing skirt 42 that is secured to the outer surfaces of the inflow end portions of the leaflets 34a, 34b, 34c at a suture line 44 adjacent the inflow end of the prosthetic valve. The inflow end portion of the leaflet assembly 14 can be secured to the stent 12 by suturing the skirt 42 to struts 16 of the lower section 24 of the stent (best shown in FIG. 1). As shown in FIG. 7, the leaflet assembly 14 can further include an inner reinforcing strip 46 that is secured to the inner surfaces of the inflow end portions 60 of the leaflets.

Referring to FIGS. 1 and 2, the outflow end portion of the leaflet assembly 14 can be secured to the upper portion of the stent 12 at three angularly spaced commissure attachments of the leaflets 34a, 34b, 34c. As best shown in FIG. 2, each commissure attachment can be formed by wrapping a reinforcing section 36 around adjacent upper edge portions 38 of a pair of leaflets at the commissure formed by the two leaflets and securing the reinforcing section 36 to the edge portions 38 with sutures 48. The sandwiched layers of the reinforcing material and leaflets can then be secured to the struts 16 of the stent 12 with sutures 50 adjacent the outflow end of the stent. The leaflets therefore desirably extend the entire length or substantially the entire length of the stent from the inflow end 26 to the outflow end 27. The reinforcing sections 36 reinforces the attachment of the leaflets to the stent so as to minimize stress concentrations at the suture lines and avoid "needle holes" on the portions of the leaflets that flex during use. The reinforcing sections 36, the skirt 42, and the inner reinforcing strip 46 desirably are made of a bio-compatible synthetic material, such as polytetrafluoroethylene (PTFE), or a woven fabric material, such as woven polyester (e.g., polyethylene terephtalate) (PET)).

FIG. 7 shows the operation of the prosthetic valve 10. During diastole, the leaflets 34a, 34b, 34c collapse to effectively close the prosthetic valve. As shown, the curved shape of the intermediate section 22 of the stent 12 defines a space between the intermediate section and the leaflets that mimics the Valsalva sinuses. Thus, when the leaflets close, backflow entering the "sinuses" creates a turbulent flow of blood along the upper surfaces of the leaflets, as indicated by arrows 52. This turbulence assists in washing the leaflets and the skirt 42 to minimize clot formation.

The prosthetic valve 10 can be implanted in a retrograde approach where the prosthetic valve, mounted in a crimped state at the distal end of a delivery apparatus, is introduced into the body via the femoral artery and advanced through the aortic arch to the heart, as further described in U.S. Patent Publication No. 2008/0065011, which is incorporated herein by reference.

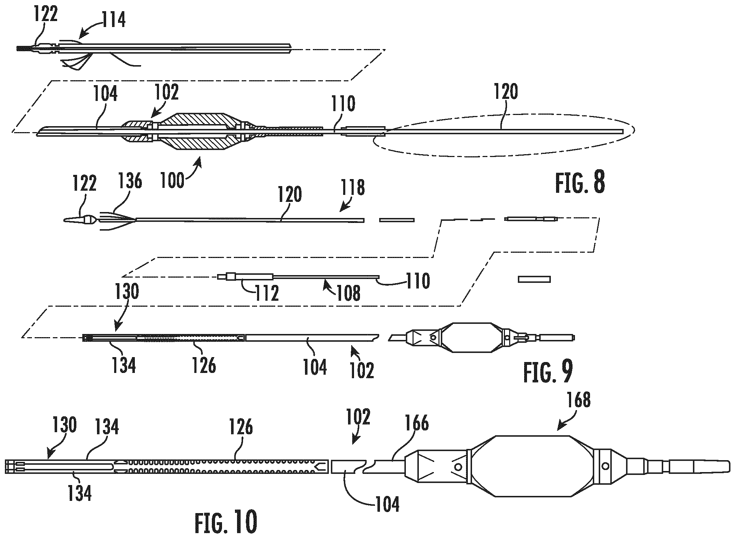

FIGS. 8-10 show a delivery apparatus 100, according to one implementation, that can be used to deliver a self-expanding prosthetic valve, such as prosthetic valve 10 described above, through a patient's vasculature. Another implementation of a delivery apparatus is described in U.S. Patent Nos. 8,652,202, 9,155,619, 9,867,700, where are incorporated herein by reference.

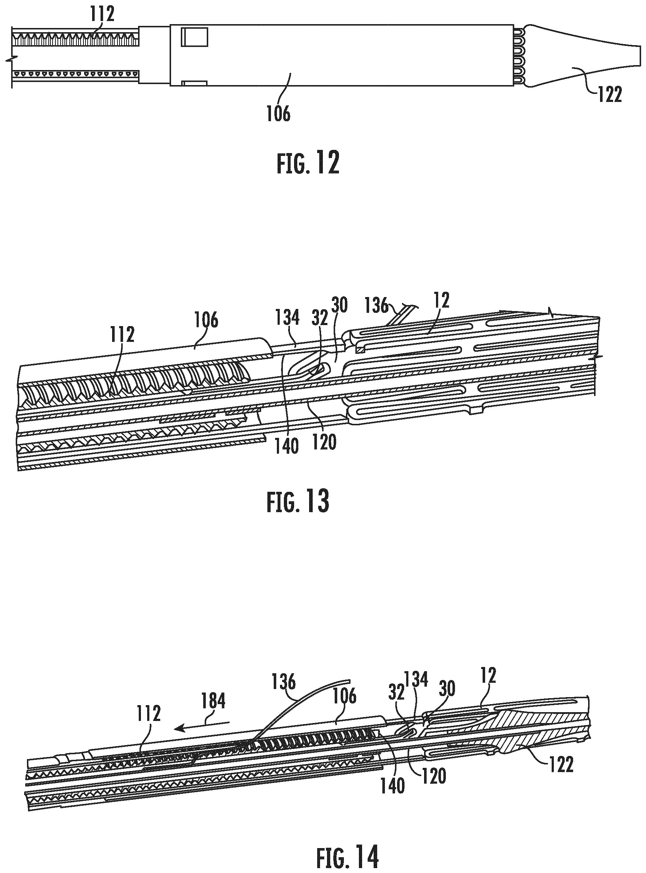

The delivery apparatus 100 comprises a first, outermost or main catheter 102 (shown alone in FIG. 10) having an elongated shaft 104, the distal end of which is coupled to a delivery sheath 106 (FIG. 12; also referred to as a delivery cylinder). The proximal end of the main catheter 102 is connected to a handle of the delivery apparatus. The handle mechanism can include an electric motor for operating the delivery apparatus. During delivery of a prosthetic valve, the handle can be used by a surgeon to advance and retract the delivery apparatus through the patient's vasculature. Although not required, the main catheter 102 can comprise a guide catheter that is configured to allow a surgeon to guide or control the amount the bending or flexing of a distal portion of the shaft 104 as it is advanced through the patient's vasculature, such as further described below. Another implementation of a guide catheter is disclosed in U.S. Patent Publication No. 2008/0065011, which is incorporated herein by reference.

As shown in FIG. 9, the delivery apparatus 100 also includes a second, intermediate catheter 108 (also referred to herein as a torque shaft catheter) having an elongated shaft 110 (also referred to herein as a torque shaft) and an elongated screw 112 connected to the distal end of the shaft 110. The shaft 110 of the intermediate catheter 108 extends coaxially through the shaft 104 of the main catheter 102. The delivery apparatus 100 can also include a third, nose-cone catheter 118 having an elongated shaft 120 and a nose piece, or nose cone, 122 secured to the distal end portion of the shaft 120. The nose piece 122 can have a tapered outer surface as shown for atraumatic tracking through the patient's vasculature. The shaft 120 of the nose-cone catheter extends through the prosthetic valve 10 (not shown in FIGS. 8-9) and the shaft 110 of the intermediate catheter 108. In the illustrated configuration, the innermost shaft 120 is configured to be moveable axially and rotatably relative to the shafts 104, 110, and the torque shaft 110 is configured to be rotatable relative to the shafts 104, 120 to effect valve deployment and release of the prosthetic valve from the delivery apparatus. For example, the torque shaft 110 is desirably configured to be rotatable relative to the delivery sheath 106 to effect incremental and controlled advancement of the prosthetic valve 10 from the delivery sheath 106.

Additionally, the innermost shaft 120 can have a lumen for receiving a guide wire so that the delivery apparatus can be advanced over the guide wire inside the patient's vasculature.

As best shown in FIG. 10, the outer catheter 102 can comprise a flex control mechanism 168 at a proximal end thereof to control the amount the bending or flexing of a distal portion of the outer shaft 104 as it is advanced through the patient's vasculature. For example, in some implementations, the flex control mechanism 168 can comprise a rotatable housing, or handle portion, 186 where rotating the housing in a first direction (e.g., clockwise), causes the distal end of the delivery apparatus to bend or flex. Rotating the housing in a second direction (e.g., counterclockwise), which relieves tension on the flex control mechanism 168 and allows the distal end of the delivery apparatus to flex back to its pre-flexed configuration under its own resiliency. The outer shaft 104 can comprise a proximal segment 166 that extends from the flex control mechanism 168 and a distal segment 126 that comprises a slotted metal tube that increases the flexibility of the outer shaft at this location. In the illustrated implementation, the proximal segment 166 extends from the flex control mechanism 168 to the distal segment 126 and therefore makes up the majority of the length of the outer shaft 104. In alternative implementations, the entire length or substantially the entire length of the outer shaft 104 can be formed from a slotted metal tube comprising one or more sections of interconnected links 160. In any case, the use of a main shaft having such a construction can allow the delivery apparatus to be highly steerable.

The distal end portion of the distal segment 126 of the outer catheter 102 can comprises an outer fork 130 of a valve-retaining mechanism 114 that is configured to releasably secure a prosthetic valve 10 to the delivery apparatus 100 during valve delivery, as described in detail below.

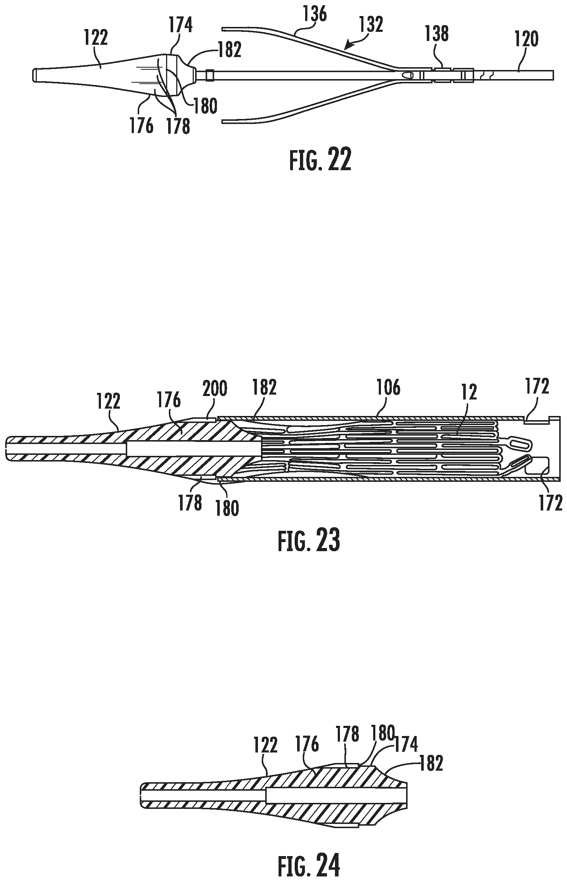

As illustrated in FIGS. 12 and 23, the sheath 106 extends over the prosthetic valve 10 and retains the prosthetic valve in a radially compressed state until the sheath 106 is retracted by the user to deploy the prosthetic valve. As described in U.S. Patent No. 9,867,700, the rotation of the torque shaft 110 (and thus the screw 112) causes the axial movement of the sheath 106 relative to the valve-retaining mechanism. Rotation of the torque shaft 110 in a first direction causes the sheath 106 to move in the proximal direction, thereby deploying the prosthetic valve from the sheath 106. Rotation of the torque shaft 110 to effect axial movement of the sheath 106 can be accomplished with a motorized mechanism or by manually turning a crank or wheel.

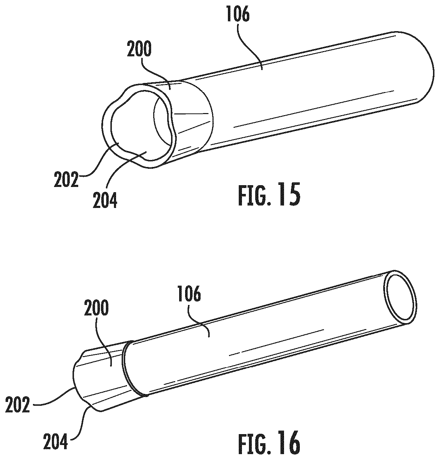

As illustrated in FIGS. 15-20 and 25-27, the sheath 106 can include a tip portion 200 provided at the distal end of the sheath 106. The tip portion 200 can define a generally cylindrical structure having a central lumen co-axial with the central lumen of the sheath 106. As will be described in more detail below, the tip portion 200 includes alternating portions of concave and convex curvature 202, 204 that extend (at least partially) longitudinally along the tip portion 200. The concave and convex portions 202, 204 facilitate retrieval and folding of the prosthetic valve 10 as it is withdrawn through the opening at the distal end of the tip portion 200 and into the sheath 106. In particular, the concave and convex portions 202, 204 are defined such that at least one cross-sectional segment of an inner surface 206 of the tip portion includes alternating portions of concave and convex curvature 202, 204 (where the concave or convex curvature is defined with respect to the longitudinal axis of the sheath 106). The concave and convex portions 202, 204 define a surface having a decreasing taper from the distal end of the tip portion 200 towards the sheath 106.

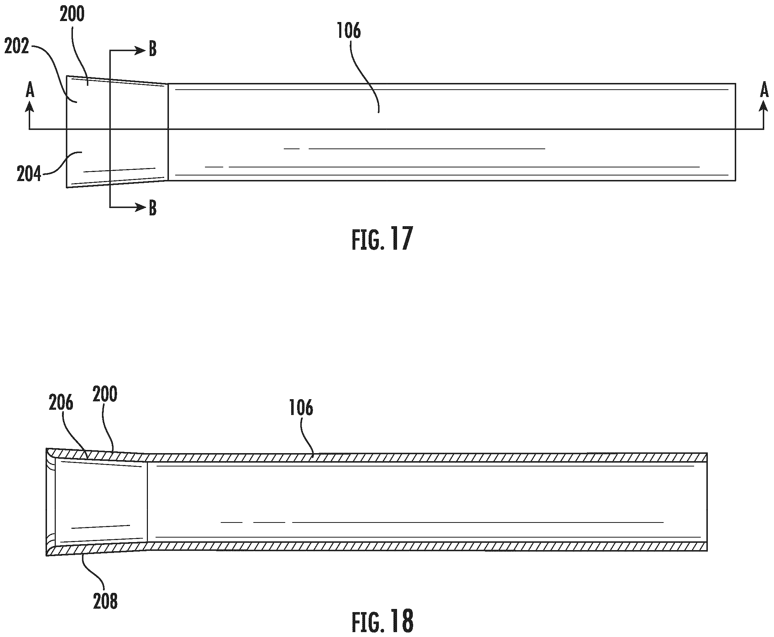

FIG. 17 provides a side view of the delivery sheath 106 and tip portion 200 and FIG. 18 provides a cross-section view of the sheath 106 and tip portion 200 of FIG. 17 along section line A-A. As illustrated in FIG. 18, diameter of the inner surface 206 at the distal end of the tip portion 200 is greater than the diameter of the inner surface 206 at the proximal end of the tip portion 200 creating a decreasing tapered surface extending between the distal and proximal ends of the tip portion 200. The tapered inner surface 206 of the tip portion 200, including the concave and convex portions 202, 204, folds or crimps the heart valve 10 as it is withdrawn into the sheath 106 and counteracting the outward radial force of the self-expanding heart valve 10, reducing the amount of force necessary to withdraw the valve (fully or partially) into the sheath 106. It is contemplated that the diameter of the inner surface 106 at the proximal end of the tip portion is at least 5% less than the diameter of the inner surface 106 at the distal end of the tip portion. In another example, the dimeter of the inner surface 106 at the proximal end of the tip portion is at least 10% less than the diameter of the inner surface 106 at the distal end of the tip portion. In a further example, the dimeter of the inner surface 106 at the proximal end of the tip portion is at least 15% less than the diameter of the inner surface 106 at the distal end of the tip portion. In yet a further example, the dimeter of the inner surface 106 at the proximal end of the tip portion is at least 20% less than the diameter of the inner surface 106 at the distal end of the tip portion. In another example, the dimeter of the inner surface 106 at the proximal end of the tip portion is no more than 25% less than the diameter of the inner surface 106 at the distal end of the tip portion.

As illustrated in FIG. 18, the diameter of the inner surface 206 of the tip portion 200 corresponds to the diameter of the inner surface of the sheath 106, ensuring the smooth transition of the heart valve 10 through the central lumen of the tip portion 200 to the into the sheath 106. It is also contemplated that a wall thickness of at least the proximal end of the tip portion 200 will correspond to the wall thickness of the sheath 106.

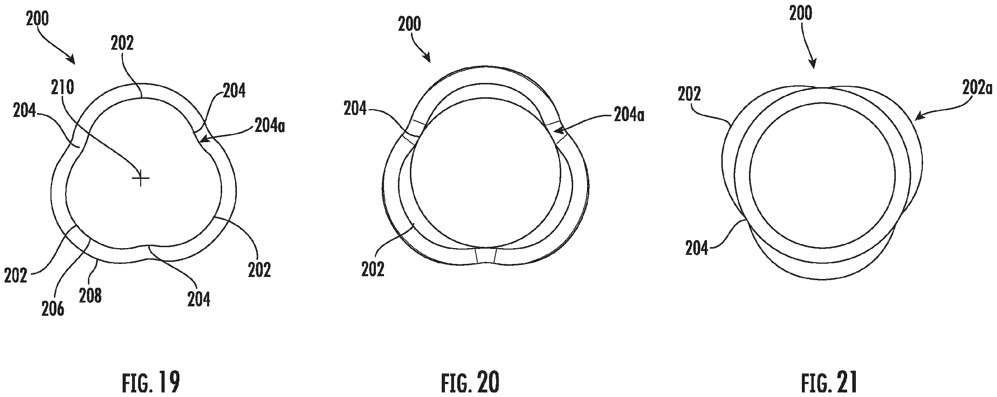

FIG. 19 is a cross-section view of the tip portion 200 along section lines B-B (FIG. 17). As provided in FIG. 19, the alternating concave and convex portions 202, 204 are formed symmetrically about the circumference of the tip portion 200. The alternating concave and convex portions 202, 204 are also distributed or spaced evenly about the circumference of the tip portion 200. It is contemplated that the tip portion 200 will have at least at least two portions of concave curvature 202 and at least two portions of convex curvature 204. While FIG. 19 illustrates a tip portion 200 having three segments of concave curvature 202 and three segments of convex curvature 204, it is contemplated that the tip portion will include no more than eight portions of concave curvature and no more than eight portions of convex curvature.

The radius of curvature of each of the concave and convex portions 202, 204 can be equal or vary around the circumference of the tip portion 200. For example, each of the concave and convex portions 202, 204 can have the same radius of curvature. Likewise, the radius of curvature of each of the concave and convex portions 202, 204 can vary around the circumference of the tip portion 200. In another example, each of the concave portions 202 may have the same radius of curvature, while each of the convex portions 204 may have the same radius of curvature (but different from the concave radius of curvature). In another example, various combination of the concave and convex portions 202, 204 have the same radius of curvature. It is also contemplated that each of the concave and convex portions 202, 204 may be equal or varying arc length, the arc length measured along the inner surface 206 of the tip portion 200 and defined as the portion of the concave or convex portions 202, 204 having a uniform radius of curvature. For example, each of the concave and convex portions 202, 204 can have the same arc length. Likewise, the arc length of each of the concave and convex portions 202, 204 can vary around the circumference of the tip portion 200. In another example, each of the concave portions 202 may have the same arc length, while each of the convex portions 204 may have the same arc length (that is different from the concave portions' arc length). In another example, various combination of the concave and convex portions 202, 204 have the same arc length.

FIGS. 20 and 21 provide distal and proximal end views of the tip portion 200 and the sheath 206. As illustrated in FIGS. 19 and 20, the apex (e.g., 204a) of the convex portions 204 defines the minimum radius of the inner surface 206 of the tip portion 200. That is, the apex 204a of at least one of the convex portions 204 defines the inner most surface of the tip portion with respect to the longitudinal axis 210 of the tip portion 200. For example, the apex 204a of at least one of the convex portions 204 adjacent the proximal end of the tip portion 200 defines the minimum radius of the inner surface 206 of the tip portion 200, as illustrated, for example in FIG. 20. In some examples, the tip portion 200 includes a cylindrically-shaped segment extending between the proximal end of the tip portion 200 and the longitudinal end of the concave and convex portions 202, 204. In this example, the minimum radius defined by the apex 204a of the convex portions 204 corresponds to the radius of a cylindrically-shaped segment of the tip portion 200 (i.e., the portion adjacent the sheath 106), as illustrate, for example in FIGS. 19, 20.

Similarly, the apex (e.g., 202a) of the concave portions 202 defines the maximum radius of the inner surface 206 of the tip portion 200. That is, the apex 202a of at least one of the concave portions 202 defines the outermost surface of the tip portion 200 with respect to the longitudinal axis 210. For example, the apex 202a of at least one of the concave portions 202 adjacent a distal end of the tip portion 200 defines the maximum radius of the inner surface 206 of the tip portion 200, as illustrated, for example, in FIG. 19, 21.

As illustrated in FIG. 19, the outer surface 208 of the tip portion 200 includes alternating portions of concave and convex curvature. The alternating portions of concave and convex curvature of the outer surface 208 are aligned (e.g., circumferentially and/or radially aligned) with the alternating portions of concave and convex curvature of the inner surface 206. Likewise, the alternating portions of concave and convex curvature of the outer surface 208 may have a radius of curvature and/or arc length corresponding or varying from the alternating concave and convex portions 202, 204 of the inner surface 206. It is contemplated that the outer surface 208 of the tip portion 200 defines a conically-shaped outer surface while maintaining the alternating concave and convex portions 202, 204 of the inner surface 206. It is further contemplated that the outer surface 208 of the tip portion 200 may define a cylindrically-shaped outer surface while maintaining the alternating concave and convex portions 202, 204 of the inner surface 206.

As illustrated in FIGS. 18 and 19, the tip portion 200 has uniform thickness both circumferentially and longitudinally. However, it is contemplated that various portions of the tip portion 200, e.g., the portion including the concave and convex portions 202, 204 may have varying circumferential and/or longitudinal thickness. The tip portion 200 can be formed from the same or different material as the sheath 106. In some examples, the tip portion 200 is formed from a semirigid polymer such as nylon. In this example, the semirigid polymer is nor more than 10% volumetrically expandable. The tip portion 200 can be formed integrally with the sheath 106 or coupled to the sheath 106 using any chemical and/or mechanical fastener known in the art. Alternatively, the tip portion 200 can be construction from a polymeric material that is reflowed or molded to the distal end portion of the sheath 106.

FIG. 22 shows an enlarged view of the nose cone 122 secured to the distal end of the innermost shaft 120. The nose cone 122 in the illustrated implementation includes a proximal end portion 174 that is sized to fit inside the tip portion 200 and the distal end of the sheath 106. An intermediate section 176 of the nose cone is positioned immediately adjacent the end of the tip portion 200 in use and is formed with a plurality of concave and convex portions 178 corresponding to the tip portion 200. The diameter of the intermediate section 176 at its proximal end 180 desirably is slightly larger than the outer diameter of the tip portion 200. The proximal end 180 can be held in close contact with the distal end of the tip portion 200 to protect surrounding tissue from coming into contact with the distal edge of the sheath/tip portion 200. The concave and convex portions of the nose cone 122 allow the intermediate section 176 to be compressed radially as the delivery apparatus is advanced through an introducer sheath. This allows the nose cone to be slightly oversized relative to the inner diameter of the introducer sheath. FIG. 24 shows a cross-section of the nose cone 122 and FIG. 23 shows a cross-section of the nose cone 122 and the sheath 106 in a delivery position with the prosthetic valve 10 retained in a compressed delivery state inside the sheath 106 (for purposes of illustration, only the stent 12 of the prosthetic valve is shown). As shown, the proximal end 180 of the intermediate section 176 can abut the distal end of the sheath 106 and a tapered proximal surface 182 of the nose cone can extend within a distal portion of the stent 12.

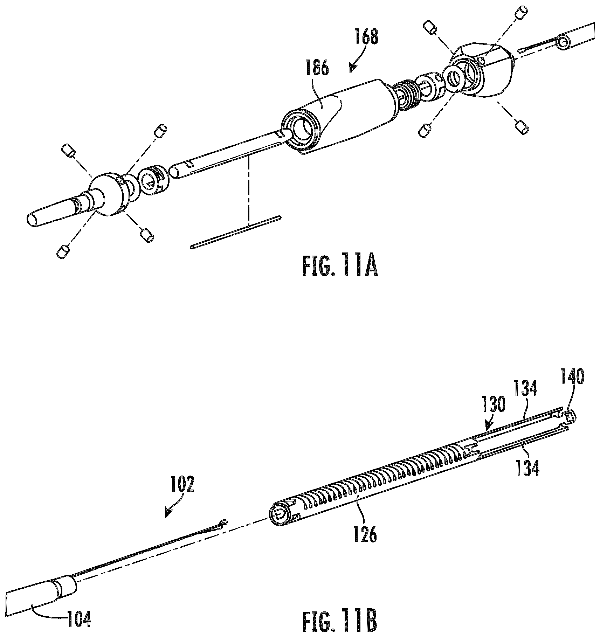

As noted above, the delivery apparatus 100 can include a valve-retaining mechanism 114 (FIG. 8) for releasably retaining a stent 12 of a prosthetic valve. The valve-retaining mechanism 114 can include a first valve-securement component in the form of an outer fork 130 (as best shown in FIG. 11B) (also referred to as an "outer trident" or "release trident"), and a second valve-securement component in the form of an inner fork 132 (as best shown in FIG. 22) (also referred to as an "inner trident" or "locking trident"). The outer fork 130 cooperates with the inner fork 132 to form a releasable connection with the retaining arms 30 of the stent 12.

The proximal end of the outer fork 130 is connected to the distal segment 126 of the outer shaft 104 and the distal end of the outer fork is releasably connected to the stent 12. In the illustrated implementation, the outer fork 130 and the distal segment 126 can be integrally formed as a single component (e.g., the outer fork and the distal segment can be laser cut or otherwise machined from a single piece of metal tubing), although these components can be separately formed and subsequently connected to each other. The inner fork 132 can be mounted on the nose catheter shaft 120 (as best shown in FIG. 22). The inner fork 132 connects the stent to the distal end portion of the nose catheter shaft 120. The nose catheter shaft 120 can be moved axially relative to the outer shaft 104 to release the prosthetic valve from the valve-retaining mechanism, as further described below.

As best shown in FIG. 11B, the outer fork 130 includes a plurality of angularly-spaced prongs 134 (three in the illustrated implementation) corresponding to the retaining arms 30 of the stent 12, which prongs extend from the distal end of distal segment 126. The distal end portion of each prong 134 includes a respective opening 140. As shown in FIG. 17, the inner fork 132 includes a plurality of angularly-spaced prongs 136 (three in the illustrated implementation) corresponding to the retaining arms 30 of the stent 12, which prongs extend from a base portion 138 at the proximal end of the inner fork. The base portion 138 of the inner fork is fixedly secured to the nose catheter shaft 120 (e.g., with a suitable adhesive) to prevent axial and rotational movement of the inner fork relative to the nose catheter shaft 120.

Each prong of the outer fork cooperates with a corresponding prong of the inner fork to form a releasable connection with a retaining arm 30 of the stent. In the illustrated implementation, for example, the distal end portion of each prong 134 is formed with an opening 140. When the prosthetic valve is secured to the delivery apparatus (as best shown in FIG. 14), each retaining arm 30 of the stent 12 extends inwardly through an opening 140 of a prong 134 of the outer fork and a prong 136 of the inner fork is inserted through the opening 32 of the retaining arm 30 so as to retain the retaining arm 30 from backing out of the opening 140. Retracting the inner prongs 136 proximally (in the direction of arrow 184 in FIG. 14) to remove the prongs from the openings 32 is effective to release the prosthetic valve 10 from the retaining mechanism. When the inner fork 132 is moved to a proximal position (FIG. 14), the retaining arms 30 of the stent can move radially outwardly from the openings 140 in the outer fork 130 under the resiliency of the stent. In this manner, the valve-retaining mechanism 114 forms a releasable connection with the prosthetic valve that is secure enough to retain the prosthetic valve relative to the delivery apparatus to allow the user to fine tune or adjust the position of the prosthetic valve after it is deployed from the delivery sheath. When the prosthetic valve is positioned at the desired implantation site, the connection between the prosthetic valve and the retaining mechanism can be released by retracting the nose catheter shaft 120 relative to the outer shaft 104 (which retracts the inner fork 132 relative to the outer fork 130).

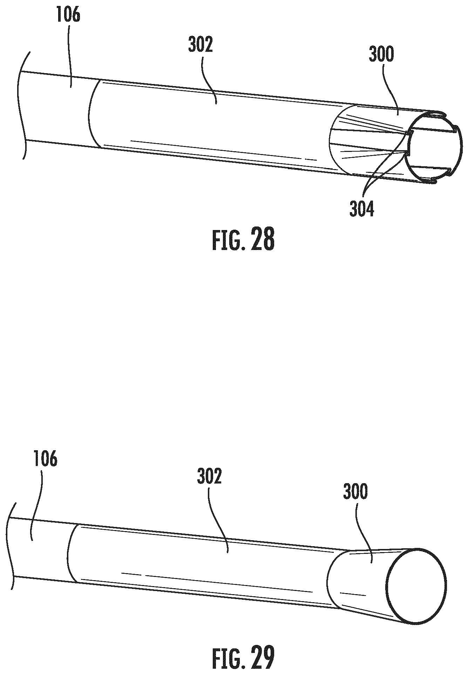

In an alternate implementation, the sheath 106 can include a tip portion 300 provided at the distal end of the sheath 106. As illustrated in FIGS. 28-30, the tip portion 300 includes longitudinally extending folding regions 304 spaced around the circumference of the tip portion 302. The folded regions 304 are movable between a folded configuration (FIG. 28) and an unfolded configuration (FIG. 29). In the unfolded configuration, the distal end of the tip portion 300 flares/expands defining an increasing tapered/conically shaped surface, with the larger diameter at the distal end of the tip portion 300.

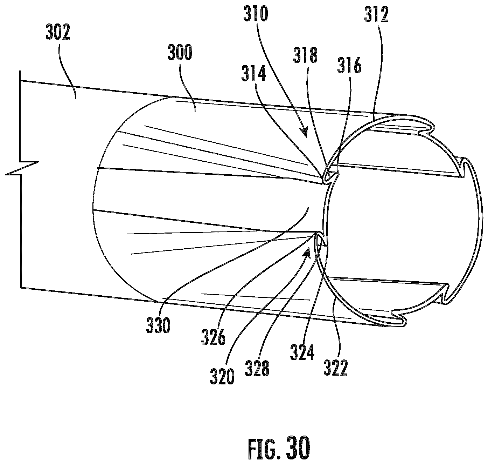

FIG. 30 is an enlarged perspective view of the distal end of the delivery sheath of FIG. 28 in the folded configuration. As described in more detail below, in the folded configuration, the tip portion 300 creases along several longitudinally extending edges such that circumferential portions of the tip portion 300 at least partially overlap. As illustrated in FIG. 30, the tip portion 300 includes a single or a plurality of folding regions 304 circumferentially spaced around the tip portion 300. The tip portion 300 can include at least two folded regions 304. For example, the tip portion 300 can include six folding regions 304 spaced around its circumference. It is contemplated that the tip portion 300 can include six or more folding regions 304. As provided in FIG. 30, the folding regions 304 can be spaced at regular intervals around the circumference of the tip portion 300. Alternatively, the folding regions 304 can be spaced at irregular intervals around the circumference of the tip portions 300. Accordingly, the folding regions 304 can be symmetrically or asymmetrically spaced around a circumference of the tip portion 300.

The structure of a first folding region 310 is defined by a first (outer) circumferential portion 312, a first and second longitudinal edges 314, 316, and a second (intermediate) circumferential portion 318 extending between the first and second longitudinal edges 314, 316. The first folding region 310 is configured to crease at the first and second longitudinal edges 314, 316 into the folded configuration such that the first and second circumferential portions 312, 318 at least partially overlap. As illustrated in FIG. 30, when folded, the second circumferential portion 318 is radially inward of the first circumferential portion 312.

Similarly, the tip portion 300 can include a second folding region 320 defined by a third (outer) circumferential portion 322, a third and fourth longitudinal edge 324, 326, and fourth (intermediate) circumferential portion 328 extending between the first and second longitudinal edge 324, 326. The second folding region 320 is configured to crease at the third and fourth longitudinal edges 324, 326 into the folded configuration such that the third and fourth circumferential portions 322, 328 at least partially overlap. As illustrated in FIG. 30, when folded, the fourth circumferential portion 328 is radially inward of the third circumferential portion 322.

As provided in FIG. 30, the first and second folding regions 310, 320 are spaced circumferentially around the tip portion 300 on opposing sides of a fifth (inner) circumferential portion 330. The fifth circumferential region 330 extends between the second longitudinal edge 316 of the first folding region 310 and the third longitudinal edge 324 of the second folding region 320. In the folded configuration, the fifth (inner) circumferential portion 330 is radially inward of the second and fourth (intermediate) circumferential portions 318, 328 (and the first and third (outer) circumferential portions 312, 322). The width of the fifth circumferential portion 320, measured around the circumference of the tip portion 300 (e.g., arc length), is less than the width of the first circumferential portion 320 and/or the third circumferential portion 322. The width of either of the second and fourth circumferential portions 316, 326 is less than the width of the fifth circumferential portion 330.

As provided in FIGS. 28 and 30, additional similarly structured folding regions 304 are spaced uniformly around the circumference of the tip portion 300. When the sheath is in the unfolded configuration, the various circumferential portions extend around the circumference of the tip portion 300 and the longitudinal edges flatten out and unfold/uncrease, defining a continuous outer perimeter of the distal end of the tip portion 300.

As will be described in more details below, in some implementations, the folding regions 304 can be biased in the folded configuration such that when there is no load applied to the inner surface of the tip portion 300 (e.g., the outward directed radial force of a passing implant), the tip portion 300 returns to the folded configuration. It is also contemplated that an outer jacket or elastomeric layer can be provided over the outer surface of the tip portion 300 to bias the folding regions 304 to return to the folded configuration.

In general, in both the folded and unfolded configurations, the tip portion 300 defines a continuous tubular structure having a central lumen extending therethrough. The diameter of the tip portion 300 in the folded configuration corresponds to a diameter of the ring portion 302 (described in more detail below). The diameter of the tip portion 300 in the unfolded configuration is greater than the diameter of the ring portion 302. The tip portion 300 and the ring portion 302 form a continuous central lumen extending between a proximal end of the ring portion 302 and a distal end of the tip portion 300. The inner diameter of the central lumen of the tip portion 300 corresponds to the inner diameter of the central lumen of the ring portion 302.

When folded, the tip portion 300 forms a cylindrical tubular structure, with the folding portions 304 formed therein, e.g., heat set. In the unfolded configuration, the tip portion 300 defines conically-shaped inner and outer surfaces. As illustrated in FIGS. 28 and 29, at least a portion of the tip portion 300 (i.e., the portion adjacent the proximal end of the tip portion 300 abutting the ring portion 302) includes a cylindrically-shaped inner and outer surface.

It is contemplated that the tip portion 300 can have a thickness corresponding to the thickness of the ring portion 302. Alternatively, the thickness of the tip portion 300 is less than the thickness of the ring portion 302. It is further contemplated that the tip portion 300 has a uniform thickness along its length. Alternatively, the tip portion 300 has a non-uniform/varying thickness along its length. Similarly, the tip portion 300 has a uniform thickness around its circumference.

As described above, the tip portion 300 expands from the folded to the unfolded configuration in response to the outward directed radial force of a passing implant/medical device. To facilitate expansion/unfolding, the tip portion 300 can have an elastic modulus less than the elastic modulus of the ring portion 302. Alternatively, the tip portion 300 has an elastic modulus corresponding to the elastic modulus of the ring portion 302.

As illustrated in FIGS. 28 and 29, a ring (holding) portion 302 can be provided between the distal end of the sheath 106 and the proximal end of the tip portion 300. The ring portion 302 can maintain the heart valve/implant in a compressed configuration before passing to the tip portion 300. The ring portion 302 defines an elongated tubular structure having a central lumen extending therethrough such that the ring portion 302 defines cylindrically-shaped inner and outer surfaces. The ring portion 302 and the sheath member 106 form a continuous central lumen extending between the proximal end of the sheath member 106 and a distal end of the ring portion 302/tip portion 300. In general, the inner diameter of the central lumen extending through the ring portion 302 corresponds to/is the same as the inner diameter of a central lumen of the sheath member 106. Similarly, the ring portion 302 and the sheath member 106 can have the same thickness (e.g., wall thickness measured radially between the inner and outer surface of the ring portion 302/sheath member 106). Alternatively, the ring portion 302 can have a wall thickness greater than the sheath member 106. It is contemplated that the ring portion 302 has a uniform thickness along its length. Similarly, the ring portion 302 has a uniform thickness around its circumference.

As described above, the ring portion 302 can maintain the heart valve in a compressed configuration before passing into the tip portion 300. In general, the elastic modulus of the ring portion 302 corresponds to or is greater than the elastic modulus of the sheath member. In another example, the ring portion 302 has an elastic modulus less than an elastic modulus of the sheath member 106.

As illustrated in FIGS. 28 and 29, the length of the ring portion 302 corresponds to or is greater than the length of the tip portion 300. For example, the length of the ring portion 302 is at least twice the length of the tip portion 300. In another example, the length of the ring portion 302 is less than the length of the tip portion 300. In general, the length of the ring portion 302 at least corresponds to the length of a compressed/crimped prosthetic heart valve.

The sheath member 106, the ring portion 302, and the tip portion 300 can be constructed from the same or different material. The ring portion 302 can be formed as a separate structure and coupled to the distal end of the sheath member 106. Similarly, the tip portion 300 can be formed as a separate structure and coupled to the distal end of the ring portion 302. Alternatively, the ring portion 302 can be integrally formed with the sheath member 106 and/or the tip portion 300.

When combined with a self-expandable prosthetic heart valve, the central lumens of both the sheath member 106 and the ring portion 302 are sized and configured to receive and maintain the prosthetic heart valve in a compressed configuration. The tip portion 300 moves from the folded to the unfolded configuration in response to the outward directed radial force of the prosthetic heart valve as it passes through the tip portion 300. Movement from the folded configuration to the unfolded configuration causes the tip portion 300 to transition from an elongated cylindrical shape to a conical shape defining an increasing tapered inner surface, where the diameter of the distal end of the tip portion 300 is greater than the diameter of the proximal end of the tip portion 300. The prosthetic heart valve can at least partially expand from a compressed configuration towards an expanded configuration as it passes through the tip portion 300, and fully expand as it passes beyond the distal end of the tip portion 300. The tip portion 300 can be biased towards the folded configuration such that the tip portion 300 moves back towards the folded configuration after the prosthetic heart valve passes through the tip portion 300.

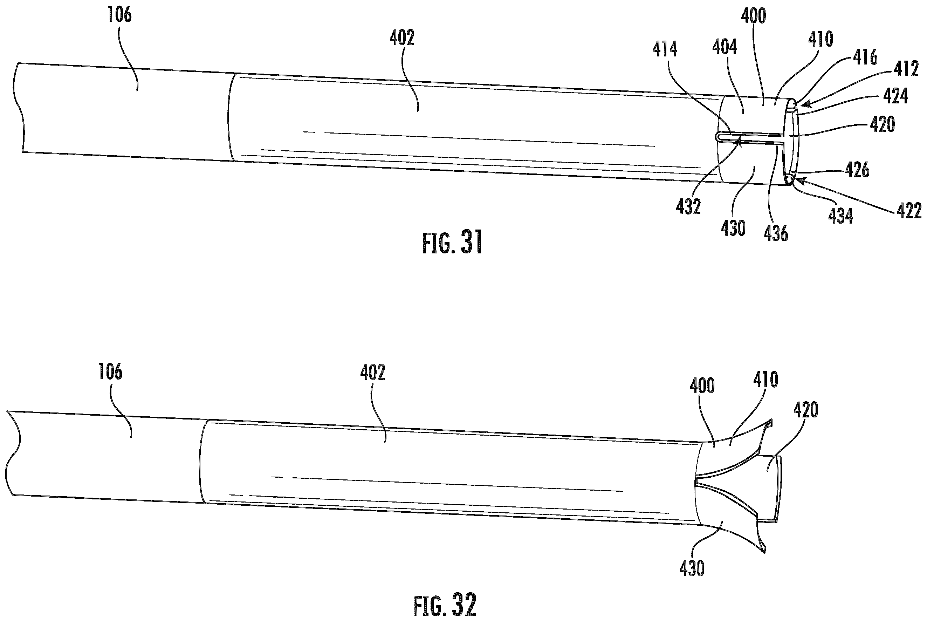

In another implementation, the sheath 106 can include a tip portion 400 provided at a distal end of the sheath 106. As illustrated in FIGS. 31-32, the tip portion 400 includes flaps 404/arms that flare open to provide a distal end of the sheath 106 having an increased diameter to accommodate passage of the implant/medical device.

When expanded the tip portion 402 is forms a discontinuous surface having two flaps 404 that extending circumferentially around the tip portion. The tip portion 400 is movable between an unexpanded (FIG. 31) and an expanded configuration (FIG. 32). In the unexpanded configuration, the tip portion 400 defines a constant diameter along its length. Whereas, in the expanded configuration, the tip portion 400 flares open increasing the diameter of the distal end of the tip portion 40 and increasing the spacing between the flaps 404.

The flaps 404 can be formed symmetrically about the tip portion 400 such that the circumferential width of the various flaps correspond. For example, as illustrated in FIG. 31, the sheath 106 can include three flaps having equal width and symmetrically spaced around the circumference of the tip portion 400. Alternatively, the flaps 404 can be formed asymmetrically about the tip portion 400 such that the circumferential width of the flaps varies.

As provided in FIGS. 31 and 32, the tip portion includes a first, second, and third flap 410, 420, 430. In the unexpanded configuration, the first, second and third flaps 410, 420, 430 are formed around the tip portion 300 with a gap 412, 422, 432 provided between adjacent flaps. For example, each of the a first, second, and third flap 410, 420, 430 include a leading edge and a trailing edge, where in the unexpanded configuration the gap 412, 422, 432 is provided between adjacent leading and trailing side edges of each of the first, second and third flaps 410, 420, 430. For example, the first flap 410 includes a leading edge 414 and a trailing edge 416, the second flap 420 includes a leading edge 424 and a trailing edge 426, and the third flap 430 includes a leading edge 434 and a trailing edge 436. The first gap 412 is formed between the trailing edge 416 of the first flap 410 and the leading edge 424 of the second flap 420. The second gap 422 is formed between the trailing edge 426 of the second flap 420 and the leading edge 434 of the third flap 430. The third gap 430 is formed between the trailing edge 436 if of the third flap 430 and the leading edge 434 of the first flap 410.

In some implementations, in the unexpanded configuration, the first, second and third flaps 410, 420, 430 are coupled to each other along weakened side edges (at the leading and trailing edges). The movement of the tip portion 400 from the unexpanded to the expanded configuration causes the first, second and third flaps 410, 420, 430 to separate along the weakened side edges to increase the spacing between adjacent flaps. An example weakened side edge is formed from a perforation, a thinned portion, a crease, or a combination thereof.

As will be described in more details below, in some implementations the flaps 404 can be biased in the unexpanded configuration such that when there is no load applied to the inner surface of the tip portion 400 (e.g., the outward directed radial force of a passing implant), the tip portion 400 returns to the unexpanded configuration. It is also contemplated that an outer jacket or elastomeric layer can be provided over the outer surface of the tip portion 400 to bias the flaps 404 to return to the unexpanded configuration.

Similar to the tip portion 300 depicted in FIGS. 28-30, the tip portion 400 is coupled to the distal end of the sheath member 106, without an intermediary ring portion 402. The diameter of the proximal end of the tip portion 400 corresponding to the diameter of a distal end of the sheath portion 106. As depicted in FIGS. 31-32, the ring portion 402 is provided between the distal end of the sheath member 106 and a proximal end of the tip portion 400. The diameter of the tip portion 400 in the unexpanded configuration corresponds to the diameter of at least one of the sheath member 106 and the ring portion 402, and the diameter of the tip portion 400 in the expanded configuration is greater than the diameter of the sheath member and/or the ring portion 402. The inner surface of the tip portion 400 at its proximal end defines a circular-shape in cross-section when the tip portion 400 is in an unexpanded configuration, i.e., the inner surface of the tip portion forms an elongated cylindrical shape when in the unexpanded configuration. The tip portion 400, the ring portion 402 and the sheath member 106 form a continuous central lumen extending between a proximal end of the sheath member and a distal end of the tip portion. In general, the unexpanded inner diameter of the central lumen extending through the tip portion 400 corresponds/is the same as the inner diameter of a central lumen of the ring portion 402. Similarly, the thickness of the tip portion 400 can corresponds to the thickness of the ring portion 402 and/or the sheath member 106. Alternatively, the thickness of the tip portion 400 can be less than the thickness of the ring portion 402 and/or the sheath member 106.

As described above, the tip portion 400 expands in response to the outward directed radial force of a passing implant/medical device. To facilitate expansion, tip portion 400 (flaps 404) can have an elastic modulus less than the elastic modulus of the ring portion 402 and/or the sheath member 106. For example, the tip portion 400 (flaps 400) can be formed from a superelastic material. In some examples, the tip portion 400 (flaps 400) is formed from a metal alloy. The metal alloy is covered with a plastic to facilitate expansion/contraction and provide a smooth less traumatic surface of the flaps with respect to patient anatomy.

It is contemplated that the tip portion 400 has a uniform thickness along its length. Similarly, the tip portion 400 has a uniform thickness around its circumference. As illustrated in FIGS. 31 and 32, the length of the tip portion 400 is less than a length of the ring portion 402. In other implementations, not shown, the length of the tip portion 400 corresponds to or is greater than the length of the ring portion 402.

Similar to the implementation depicted in FIGS. 28-30, the sheath 106 includes a ring (holding) portion 402 between the distal end of the sheath 106 and the proximal end of the tip portion 402. The ring portion 402 maintains the heart valve in a compressed configuration before passing to the tip portion 400. Unless otherwise noted, the ring portion 402 includes similar structure to the ring portion 302.

The ring portion 402 can be formed as a separate structure and coupled to the distal end of the sheath member 106. Similarly, the tip portion 400 can be formed as a separate structure and coupled to the distal end of the ring portion 402. Alternatively, the ring portion 402 is integrally formed with the sheath member 106 and/or the tip portion 400.

When combined with a self-expandable prosthetic heart valve, the central lumens of both the sheath member 106 and the ring portion 402 are sized and configured to receive and maintain the prosthetic heart valve in a compressed configuration. The tip portion 400 moves from the unexpanded to the expanded configuration in response to the outward directed radial force of the prosthetic heart valve as it passes through the tip portion 400. Movement from the unexpanded configuration to the expanded configuration cause the flaps 404 to flare open such that the diameter of the distal end of the tip portion 400 is greater than a diameter of a proximal end of the tip portion 400. The prosthetic heart valve can at least partially expand from a compressed configuration towards an expanded configuration as it passes through the tip portion 400, and fully expand as it passes beyond the distal end of the tip portion 400. The tip portion 400 is biased towards the unexpanded configuration such that the tip portion 400 moves back towards the unexpanded configuration after the prosthetic heart valve passes through the tip portion 400.

Techniques for compressing and loading the prosthetic valve 10 into the sheath 106 are described below. Once the prosthetic valve 10 is loaded in the delivery sheath 106, the delivery apparatus 100 can be inserted into the patient's body for delivery of the prosthetic valve. In one approach, the prosthetic valve can be delivered in a retrograde procedure where delivery apparatus is inserted into a femoral artery and advanced through the patient's vasculature to the heart. Prior to insertion of the delivery apparatus, an introducer sheath can be inserted into the femoral artery followed by a guide wire, which is advanced through the patient's vasculature through the aorta and into the left ventricle. The delivery apparatus 100 can then be inserted through the introducer sheath and advanced over the guide wire until the distal end portion of the delivery apparatus containing the prosthetic valve 10 is advanced to a location adjacent to or within the native aortic valve.

Thereafter, the prosthetic valve 10 can be deployed from the delivery apparatus 100 by rotating the torque shaft 110 relative to the outer shaft 104. The proximal end of the torque shaft 110 can be operatively connected to a manually rotatable handle portion or a motorized mechanism that allows the surgeon to effect rotation of the torque shaft 110 relative to the outer shaft 104. Rotation of the torque shaft 110 causes the sheath 106 to move in the proximal direction toward the outer shaft, which deploys the prosthetic valve from the sheath 106. Rotation of the torque shaft 110 causes the sheath to move relative to the prosthetic valve in a precise and controlled manner as the prosthetic valve advances from the open distal end of the delivery sheath and begins to expand. Hence, unlike known delivery apparatuses, as the prosthetic valve begins to advance from the delivery sheath and expand, the prosthetic valve is held against uncontrolled movement from the sheath caused by the expansion force of the prosthetic valve against the distal end of the sheath. In addition, as the sheath 106 is retracted, the prosthetic valve 10 is retained in a stationary position relative to the ends of the inner shaft 120 and the outer shaft 104 by virtue of the valve-retaining mechanism 114. As such, the prosthetic valve 10 can be held stationary relative to the target location in the body as the sheath is retracted. Moreover, after the prosthetic valve is partially advanced from the sheath, it may be desirable to retract the prosthetic valve back into the sheath, for example, to reposition the prosthetic valve or to withdraw the prosthetic valve entirely from the body. The partially deployed prosthetic valve can be retracted back into the sheath by reversing the rotation of the torque shaft, which causes the sheath 106 to advance back over the prosthetic valve in the distal direction.

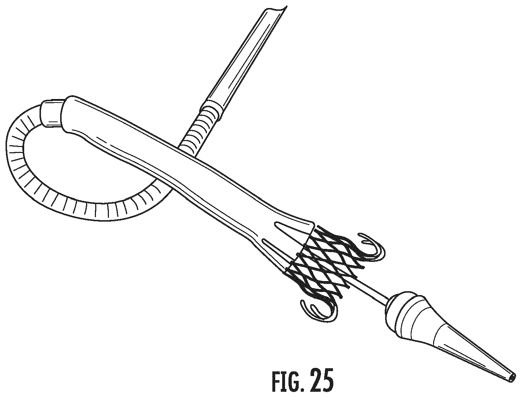

The prosthetic valve 10 advances through the distal end of the delivery sheath 106 and through the tip portion 200. The prosthetic valve 10 partially expands within the tip portion 200 as illustrated, for example, in FIG. 25. Traditional methods of deploying a self-expanding valve utilize a cylindrically-shaped sheath. However, during deployment this structure can cause the valve to "jump" and spring open rapidly in the patient anatomy, causing damage to the valve and the trauma to the patient. In contrast, when using the sheath 106 and tip portion 200 illustrated in FIGS. 15-20 and 25-27, the tapered inner surface 206 and the concave and convex portions 202, 204 of the present disclosure facilitate the controlled and directed expansion of the prosthetic valve 10. For example, the increasing taper of the tip portion 200 provides for the controlled and gradual expansion of the prosthetic valve 10. Likewise, the alternating concave and convex portions 202, 204 provide for areas within the tip of increased diameter, directing the prosthetic valve 10 to expand first towards the concave portions 202. By spacing the concave and convex portions 202, 204 symmetrically around the tip portion 200 ensures the prosthetic valve expands uniformly around its circumference. The controlled expansion of the prosthetic valve 10 is illustrated, for example, in FIGS. 25-27.

After the prosthetic valve 10 is advanced through the tip portion 200 (and at least partially expands therein) and exits beyond the distal end of the tip portion/delivery sheath, the prosthetic valve 10 expands to its functional size. The prosthetic valve remains connected to the delivery apparatus via the retaining mechanism 114. Consequently, after the prosthetic valve is advanced from the delivery sheath, the surgeon can reposition the prosthetic valve relative to the desired implantation position in the native valve such as by moving the delivery apparatus in the proximal and distal directions or side to side, or rotating the delivery apparatus, which causes corresponding movement of the prosthetic valve 10. The retaining mechanism 114 desirably provides a connection between the prosthetic valve and the delivery apparatus that is secure and rigid enough to retain the position of the prosthetic valve relative to the delivery apparatus against the flow of the blood as the position of the prosthetic valve is adjusted relative to the desired implantation position in the native valve.

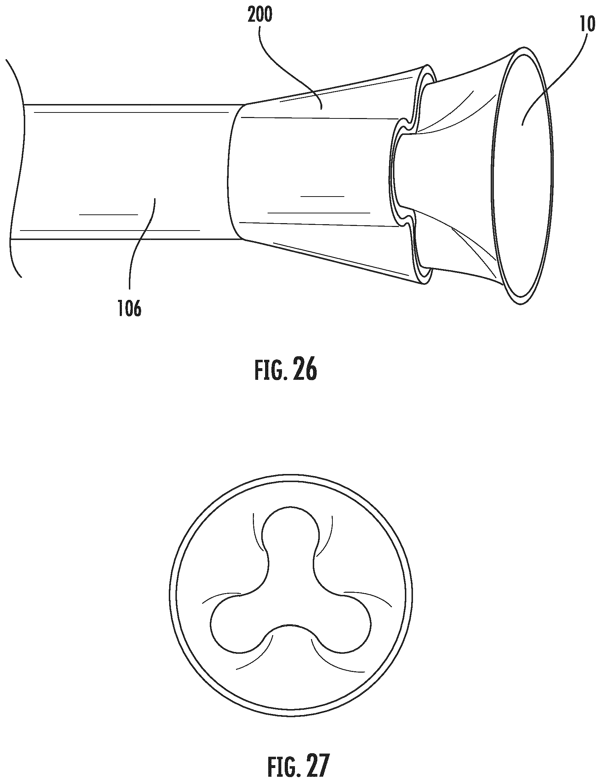

If it is necessary to retrieve the prosthetic valve 10 such as for removal or repositioning, the retaining mechanism 114 is drawn proximally and the prosthetic valve 10 is withdrawn (e.g., fully or partially) into the tip portion 200. The alternating portions of concave and convex curvature 202, 204 facilitate the directed compression (e.g., folding and/or crimping) of the prosthetic heart valve as it is withdrawn into the tip portion 200 and towards the sheath 106 (see e.g., FIGS. 25-27). FIG. 26 provides a schematic representation of the prosthetic valve 10 being drawn into the tip portion 200 and FIG.

27 provides a distal end view of the prosthetic valve 10 partially compressed within the tip portion 200. As illustrated in FIGS. 26 and 27, the portions of concave and convex curvature 202, 204 work in combination direct the uniform compression of the prosthetic valve 10. As described above, while traditional strategies for deploying and re-sheathing a self-expanding prosthetic valve utilize a cylindrical sheath to contain and deploy the valve, the large radial forces of the valve can cause it to fold inwards on itself in non-uniform ways. As a result, the valve can fold and crumple inwards irregularly, and get stuck in the sheath or even be damaged to that it cannot be re-deployed. In contrast, the alternating concave and convex portions 202, 204 provided in the present tip portion 200 creates several small arches rather than one large arch for a circular sheath. These smaller arches reduce the hoop stress in the valve, making it more stable and less likely to fold inwards. This is demonstrated in the circular arch stress equation:

$$R = \frac{wL}{2}$$

, where R is the load at the base supports, w is the applied weight, L is the span or diameter of the arch. Assuming all variables other variables are equal, decreasing the inner cylinder diameter $L$ will reduce the load on the arch R. As a result, the concave and convex curvature 202, 204 work in combination to reduce the hoop stress on the valve and provide for uniform compression of the prosthetic valve 10. Accordingly, as the prosthetic valve 10 is compressed in remains generally circular as it is compressed within the tip portion 200 and drawn into the sheath 106. The concave and convex portions 202, 204 prevent the irregular folding/crimping of the prosthetic valve 10 as it transitions between a fully/partially expanded configuration to a non/less expanded configuration.

Once the surgeon positions the prosthetic valve at the desired implantation position in the native valve, the connection between the prosthetic valve and the delivery apparatus can be released by retracting the innermost shaft 120 in the proximal direction relative to the outer shaft 104, which is effective to retract the inner fork 132 to withdraw its prongs 136 from the openings 32 in the retaining arms 30 of the prosthetic valve (FIG. 14). Slightly retracting of the outer shaft 104 allows the outer fork 130 to back off the retaining arms 30 of the prosthetic valve, which slide outwardly through openings 140 in the outer fork to completely disconnect the prosthetic valve from the retaining mechanism 114. Thereafter, the delivery apparatus can be withdrawn from the body, leaving the prosthetic aortic valve 10 implanted within the native valve (such as shown in FIGS. 5A and 5B).

In an alternative implementation, when the sheath 106 and tip portion 300 of FIGS. 28-30 is used, the prosthetic valve 10 is advanced through the distal end of the sheath member 106 into the ring portion 302. The prosthetic valve 10 is then advanced, in a compressed configuration, through the ring portion 302 into the tip portion 300. The prosthetic valve 10 at least partially expands within the tip portion 300 and the tip portion 300 moves from the folded to the unfolded configuration in response to the outward directed radial force of the prosthetic heart valve 10 passing through the tip portion 300. Advancing the prosthetic valve 10 through the tip portion 300 causes the first and second circumferential portions 312, 318 of the first folding region 310 to move to a less overlapping position than when compared to the folded configuration. Further advancing prosthetic valve 10 through the tip portion 300 causes the first and second circumferential portions 312, 318 to circumferentially align when the tip portion 300 is a fully unfolded configuration. The prosthetic valve 10 is then advanced beyond a distal end of the tip portion 300 to the treatment site. The tip portion 300 is biased towards a folded configuration such that the tip portion 300 returns toward the folded configuration after the prosthetic heart valve 10 has fully passed through the tip portion 300.

Withdrawing the prosthetic valve 10 from the treatment site is accomplished by moving the prosthetic valve 10 in a direction towards the sheath member 106 such that the prosthetic valve 10 is withdrawn (e.g., fully or partially) into the tip portion 300. The prosthetic valve 10 is disposed in an at least a partially expanded configuration when outside of the delivery sheath 106/tip portion 300. The tapered inner surface of the tip portion 300 facilitates the compression (e.g., folding and/or crimping) of the prosthetic heart valve 10 as it is withdrawn into the tip portion 300 and towards the sheath member 106. When withdrawing the prosthetic valve 10 from the treatment site further, the prosthetic valve 10 is retained in the sheath member 106 and/or the ring portion 300, the delivery sheath 106 removed from the blood vessel, and delivery sheath 106 from the introducer sheath.

In an alternative implementation, when the sheath 106 and tip portion 400 of FIGS. 31 and 32 is used, the prosthetic valve 10 is advanced through the distal end of the sheath member 106 into the ring portion 402. The prosthetic valve 10 is then advanced, in a compressed configuration, through the ring portion 402 into the tip portion 400. The prosthetic valve 10 at least partially expands within the tip portion 400 and the tip portion 400 moves from the unexpanded to the expanded configuration in response to the outward directed radial force of the prosthetic heart valve 10 passing through the tip portion 400, flaring open the tip portion 400 increasing a diameter of the distal end of the tip portion 400. Advancing the prosthetic valve 10 through the tip portion 400 causes the gaps 412, 422, 432 between the adjacent flaps to increase, increasing the spacing between flaps 404. In some implementations, in the unexpanded configuration, the flaps 404 are coupled to each other along weakened side edges. Advancing the prosthetic valve 10 through the tip portion 400 causes the flaps 404 to separate along the weakened side edges to increase the spacing between adjacent flaps 404. The prosthetic valve 10 is then advanced beyond a distal end of the tip portion 400 to the treatment site. The tip portion 400 is biased towards the unexpanded configuration such that the tip portion 400 returns toward the unexpanded configuration after the prosthetic heart valve 10 has fully passed through the tip portion 400.

Withdrawing the prosthetic valve 10 from the treatment site is accomplished by moving the prosthetic valve 10 in a direction towards the sheath member 106 such that the prosthetic valve 10 is withdrawn (e.g., fully or partially) into the tip portion 400. The prosthetic valve 10 is disposed in an at least a partially expanded configuration when outside of the delivery sheath 106/tip portion 400. The tapered inner surface of the tip portion 400 facilitates the compression (e.g., folding and/or crimping) of the prosthetic heart valve 10 as it is withdrawn into the tip portion 400 and towards the sheath member 106. When withdrawing the prosthetic valve 10 from the treatment site further, the prosthetic valve 10 is retained in the sheath member 106 and/or the ring portion 400, the delivery sheath 106 removed from the blood vessel, and delivery sheath 106 from the introducer sheath.

In an alternative implementation, the delivery apparatus can be adapted to deliver a balloon-expandable prosthetic valve. As described above, the valve retaining mechanism 114 can be used to secure the prosthetic valve to the end of the delivery apparatus. Since the stent of the prosthetic valve is not self-expanding, the sheath 106 can be optional. The retaining mechanism 114 enhances the pushability of the delivery apparatus and prosthetic valve assembly through an introducer sheath.

General Considerations

The following description of certain examples of the inventive concepts should not be used to limit the scope of the claims. Other examples, features, aspects, implementations, and advantages will become apparent to those skilled in the art from the following description. As will be realized, the device and/or methods are capable of other different and obvious aspects, all without departing from the spirit of the inventive concepts. Accordingly, the drawings and descriptions should be regarded as illustrative in nature and not restrictive.

For purposes of this description, certain aspects, advantages, and novel features of the implementations of this disclosure are described herein. The described methods, systems, and apparatus should not be construed as limiting in any way. Instead, the present disclosure is directed toward all novel and nonobvious features and aspects of the various disclosed implementations, alone and in various combinations and sub-combinations with one another. The disclosed methods, systems, and apparatus are not limited to any specific aspect, feature, or combination thereof, nor do the disclosed methods, systems, and apparatus require that any one or more specific advantages be present or problems be solved.

Features, integers, characteristics, compounds, chemical moieties, or groups described in conjunction with a particular aspect, implementation or example of the invention are to be understood to be applicable to any other aspect, implementation or example described herein unless incompatible therewith. All of the features disclosed in this specification (including any accompanying claims, abstract, and drawings), and/or all of the steps of any method or process so disclosed, may be combined in any combination, except combinations where at least some of such features and/or steps are mutually exclusive. The invention is not restricted to the details of any foregoing implementations. The invention extends to any novel one, or any novel combination, of the features disclosed in this specification (including any accompanying claims, abstract, and drawings), or to any novel one, or any novel combination, of the steps of any method or process so disclosed.

It should be appreciated that any patent, publication, or other disclosure material, in whole or in part, that is said to be incorporated by reference herein is incorporated herein only to the extent that the incorporated material does not conflict with existing definitions, statements, or other disclosure material set forth in this disclosure. As such, and to the extent necessary, the disclosure as explicitly set forth herein supersedes any conflicting material incorporated herein by reference. Any material, or portion thereof, that is said to be incorporated by reference herein, but which conflicts with existing definitions, statements, or other disclosure material set forth herein will only be incorporated to the extent that no conflict arises between that incorporated material and the existing disclosure material.

As used in the specification and the appended claims, the singular forms "a," "an" and "the" include plural referents unless the context clearly dictates otherwise. Ranges may be expressed herein as from "about" one particular value, and/or to "about" another particular value. When such a range is expressed, another aspect includes from the one particular value and/or to the other particular value. Similarly, when values are expressed as approximations, by use of the antecedent "about," it will be understood that the particular value forms another aspect. It will be further understood that the endpoints of each of the ranges are significant both in relation to the other endpoint, and independently of the other endpoint.

"Optional" or "optionally" means that the subsequently described event or circumstance may or may not occur, and that the description includes instances where said event or circumstance occurs and instances where it does not.

Throughout the description and claims of this specification, the word "comprise" and variations of the word, such as "comprising" and "comprises," means "including but not limited to," and is not intended to exclude, for example, other additives, components, integers or steps. "Exemplary" means "an example of" and is not intended to convey an indication of a preferred or ideal aspect. "Such as" is not used in a restrictive sense, but for explanatory purposes.

The terms "proximal" and "distal" as used herein refer to regions of a sheath, catheter, or delivery assembly. "Proximal" means that region closest to handle of the device, while "distal" means that region farthest away from the handle of the device.

The term "tube" or "tubular" as used herein is not meant to limit shapes to circular cross-sections. Instead, tube or tubular can refer to any elongate structure with a closed-cross section and lumen extending axially therethrough. A tube may also have some selectively located slits or openings therein - although it still will provide enough of a closed structure to contain other components within its lumen(s).

Beyond transcatheter heart valves, the expandable sheath 10 can be useful for other types of minimally invasive procedure, such as any procedure requiring introduction of an apparatus into a subject's vessel. For example, the expandable sheath 10 can be used to introduce other types of delivery apparatus for placing various types of intraluminal devices (e.g., stents, stented grafts, balloon catheters for angioplasty procedures, etc.) into many types of vascular and non-vascular body lumens (e.g., veins, arteries, esophagus, ducts of the biliary tree, intestine, urethra, fallopian tube, other endocrine or exocrine ducts, etc.).

Although the foregoing implementations of the present disclosure have been described in some detail by way of illustration and example for purposes of clarity and understanding, it will be apparent to those skilled in the art that certain changes and modifications may be practiced within the spirit and scope of the present disclosure. It should be recognized that the illustrated implementations are only preferred examples of the invention and should not be taken as limiting the scope of the invention. Moreover, additional implementations are disclosed in U.S. Patent Application Publication No. 2010/0049313 (U.S. application Ser. No. 12/429,040), and U.S. Patent Nos. 8,652,202, 9,155,619, 9,867,700, which are incorporated herein by reference. It is intended that the scope of the present invention herein disclosed should not be limited by the particular disclosed implementations described above, but should be determined only by a fair reading of the claims that follow.

**EXEMPLARY ASPECTS:**

Example 1: A delivery sheath system comprising: a sheath member defining a tubular structure extending between a proximal end and a distal end of the sheath member; a tip portion provided at the distal end of the sheath member, the tip portion having a central lumen extending therethrough, wherein at least one cross-sectional segment of an inner surface of the tip portion includes alternating portions of concave and convex curvature.

Example 2: The delivery sheath according to any example herein, particularly example 1, wherein the alternating portions of concave and convex curvature are formed symmetrically about a circumference of the tip portion.

Example 3: The delivery sheath according to any example herein, particularly examples 1 and 2, wherein the alternating portions of concave and convex curvature are distributed evenly about a circumference of the tip portion.

Example 4: The delivery sheath according to any example herein, particularly examples 1-3, wherein a radius of curvature of the portion of concave curvature is equal to a radius of curvature of the portion of convex curvature.

Example 5: The delivery sheath according to any example herein, particularly examples 1-4, wherein a radius of curvature of the portion of concave curvature is greater than a radius curvature of the portion of convex curvature.

Example 6: The delivery sheath according to any example herein, particularly examples 1-5, wherein a radius of curvature of the portion of concave curvature is less than a radius of curvature of the portion of convex curvature.

Example 7: The delivery sheath according to any example herein, particularly examples 1-6, including at least two portions of concave curvature and at least two portions of convex curvature.

Example 8: The delivery sheath according to any example herein, particularly examples 1-7, including three portions of concave curvature and three portions of convex curvature.

Example 9: The delivery sheath according to any example herein, particularly examples 1-8, including no more than eight portions of concave curvature and no more than eight portions of convex curvature.

Example 10: The delivery sheath according to any example herein, particularly examples 1-9, wherein a proximal end of the tip portion is coupled to the distal end of the sheath member, and wherein a diameter of the proximal end of the tip portion corresponds to a diameter of a distal end of the sheath portion.

Example 11: The delivery sheath according to any example herein, particularly examples 1-10, wherein the portions of concave and convex curvature extend longitudinally along the tip portion.

Example 12: The delivery sheath according to any example herein, particularly examples 1-11, wherein the portions of concave and convex curvature extend longitudinally along the tip portion and define a decreasing tapered surface extending in a longitudinal direction between the distal and proximal ends of the tip portion.

Example 13: The delivery sheath according to any example herein, particularly examples 1-12, wherein an apex of at least one of the portions of convex curvature (e.g., adjacent the proximal end of the tip portion) defines a minimum radius of the inner surface of the tip portion.

Example 14: The delivery sheath according to any example herein, particularly examples 1-13, wherein an apex of at least one of the portions of concave curvature (e.g., adjacent the distal end of the tip portion) defines a maximum radius of the inner surface of the tip portion.

Example 15: The delivery sheath according to any example herein, particularly example 14, wherein a radius of the proximal end of the tip portion is no more than twenty-five percent smaller than the maximum radius of the inner surface of the tip portion.

Example 16: The delivery sheath according to any example herein, particularly examples 1-15, wherein a proximal end of the tip portion is provided adjacent to the distal end of the sheath member, and wherein the inner surface of the tip portion at the proximal end defines a circular-shape in cross-section.

Example 17: The delivery sheath according to any example herein, particularly examples 1-16, wherein the tip portion is formed from a semirigid polymer.

Example 18: The delivery sheath according to any example herein, particularly example 17, wherein the semirigid polymer is no more than ten percent volumetrically expandable.

Example 19: The delivery sheath according to any example herein, particularly examples 1-18, wherein the tip portion further comprises an outer surface, wherein at least one cross-sectional segment of the outer surface includes alternating portions of concave and convex curvature, wherein the alternating portions of concave and convex curvature of the outer surface are aligned (e.g., circumferentially and/or radially aligned) with the alternating portions of concave and convex curvature of the inner surface.

Example 20: The delivery sheath according to any example herein, particularly examples 1-19, wherein the tip portion has a uniform thickness.

Example 21: The delivery sheath according to any example herein, particularly examples 1-18, wherein the tip portion further comprises a cylindrically-shaped outer surface.

Example 22: The delivery sheath according to any example herein, particularly examples 1-18, wherein the tip portion further comprises a conically-shaped outer surface.

Example 23: The delivery sheath according to any example herein, particularly examples 1-22, further comprising a nose cone, having a proximal end and a tapering distal end and an intermediate section therebetween, wherein the proximal end of the nose cone is detachably coupled to the distal end of the tip portion.

Example 24: The delivery sheath according to any example herein, particularly example 23, wherein at least a portion of an outer surface of the intermediate section of the nose cone includes alternating portions of concave and convex curvature corresponding to the inner surface of the tip portion.

Example 25: The delivery sheath according to any example herein, particularly examples 1-20, wherein the sheath member and the tip portion form a continuous central lumen extending between the proximal end of the sheath member and a distal end of the tip portion.

Example 26: The delivery sheath according to any example herein, particularly examples 1-25, wherein the tip portion is formed from a uniform tubular structure.

Example 27: The delivery sheath according to any example herein, particularly examples 1-25, wherein the tip portion is formed as a separate structure and coupled to the distal end of the catheter member.

Example 28: The delivery sheath according to any example herein, particularly examples 1-26, further comprising a self-expandable prosthetic heart valve, wherein a central lumen of the sheath member is sized and configured to receive the prosthetic heart valve in a compressed configuration, wherein the alternating portions of concave and convex curvature facilitate directed expansion of the prosthetic heart valve as it passes through an opening at the distal end of the tip portion, and wherein the alternating portions of concave and convex curvature facilitate retrieval (e.g., directed folding and/or crimping) of the prosthetic heart valve as it is withdrawn through the opening at the distal end of the tip portion and into the sheath member.

Example 29: A method of deploying a prosthetic valve comprising: inserting an introducer sheath into a blood vessel, inserting a delivery sheath into a central lumen of the introducer sheath, with a prosthetic valve disposed therein, the delivery sheath comprising: a sheath member defining a tubular structure extending between a proximal end and a distal end of the sheath member; a tip portion provided at the distal end of the sheath member, the tip portion having a central lumen extending therethrough, wherein at least one cross-sectional segment of an inner surface of the tip portion includes alternating portions of concave and convex curvature; advancing a prosthetic valve through the distal end of the sheath member, to the tip portion; advancing the prosthetic valve through the tip portion to a treatment site such that the prosthetic valve at least partially expands within the tip portion, wherein the alternating portions of concave and convex curvature facilitate directed expansion of the prosthetic heart valve as it passes through the tip portion.

Example 30: The method according to any example herein, particularly example 29, wherein the delivery sheath further comprises, a nose cone, having a proximal end and a tapering distal end, the nose cone detachably coupled to the distal end of the tip portion; the method further comprising advancing the nose cone away from the distal end of the tip portion.

Example 31: The method according to any example herein, particularly examples 29 and 30, further comprising withdrawing the prosthetic valve from the treatment site by moving the prosthetic valve in a direction towards the sheath member such that the prosthetic valve is withdrawn (e.g., fully or partially) into the tip portion, wherein the prosthetic valve is disposed in an at least a partially expanded configuration when outside of the delivery sheath, and wherein the alternating portions of concave and convex curvature facilitate directed compression (e.g., folding and/or crimping) of the prosthetic heart valve as it is withdrawn into the tip portion and towards the sheath member.

Example 32: The method according to any example herein, particularly examples 29-31, wherein sections of the prosthetic valve are compressed at circumferential locations corresponding to the portions of convex curvature (e.g., compressed greater at the circumferential locations corresponding to the portions of convex curvature than compared to compression of the valve at those portions of concave curvature).

Example 33: The method according to any example herein, particularly examples 29-32, wherein the prosthetic valve is a super elastic self-expanding valve.

Example 34: The method according to any example herein, particularly examples 29-33, further comprising completely removing the prosthetic valve from the tip portion.

Example 35: The method according to any example herein, particularly examples 29-34, further comprising retaining the prosthetic valve in the sheath member, removing the delivery sheath from the blood vessel, and removing the delivery sheath from the introducer sheath.

Example 36: The method according to any example herein, particularly examples 29-35, wherein the prosthetic valve is an Aortic valve.

Example 37: A delivery sheath system comprising: a sheath member defining a tubular structure extending between a proximal end and a distal end of the sheath member; a ring portion provided at the distal end of the sheath member; and a tip portion extending from a distal end of the ring portion, the tip portion including a longitudinally extending folding region movable between a folded and unfolded configuration, in the folded configuration the tip portion creases along a longitudinally extending edge such that circumferential portions of the tip portion at least partially overlap, wherein, in the unfolded configuration, the tip portion defines an increasing tapered surface having a larger diameter at a distal end of the tip portion.

Example 38: The delivery sheath according to any example herein, particularly example 37, wherein the tip portion includes a plurality of folding regions circumferentially spaced around the tip portion.

Example 39: The delivery sheath according to any example herein, particularly example 38, wherein the plurality of folding region are asymmetrically spaced around a circumference of the tip portion.

Example 40: The delivery sheath according to any example herein, particularly example 38, wherein the plurality of folding region are symmetrically spaced around a circumference of the tip portion.

Example 41: The delivery sheath according to any example herein, particularly examples 37-40, wherein the plurality of folding regions include a first folding region defined by a first (outer) circumferential portion, a first and second longitudinal edges, and a second (intermediate) circumferential portion extending between the first and second longitudinal edges, wherein the first folding region is configured to crease at the first and second longitudinal edges into the folded configuration such that the first and second circumferential portions at least partially overlap, and the second circumferential portion is radially inward of the first circumferential portion.

Example 42: The delivery sheath according to any example herein, particularly examples 37-41, wherein the plurality of folding regions include a second folding region defined by a third (outer) circumferential portion, a third and fourth longitudinal edge, and a fourth (intermediate) circumferential portion extending between the third and fourth longitudinal edges, wherein the second folding region is configured to crease at the third and fourth longitudinal edges into the folded configuration such that the third and fourth circumferential portions at least partially overlap, and the fourth circumferential portion is radially inward of the third circumferential portion.

Example 43: The delivery sheath according to any example herein, particularly examples 41-42, wherein the first folding region and the second folding region are spaced circumferentially around the tip portion on opposing sides of a fifth circumferential region.

Example 44: The delivery sheath according to any example herein, particularly example 43, wherein the fifth circumferential region extends between the second and third longitudinal edges.

Example 45: The delivery sheath according to any example herein, particularly examples 43 and 44, wherein, in the folded configuration, the fifth circumferential portion is radially inward of the second and fourth circumferential portions.

Example 46: The delivery sheath according to any example herein, particularly examples 43-45, wherein a width of the fifth circumferential portion measured around the circumference of the tip portion is less than a width of the first and third circumferential portions.

Example 47: The delivery sheath according to any example herein, particularly examples 43-46, wherein a width of either of the second and fourth circumferential portions is less than the width of the fifth circumferential portion.

Example 48: The delivery sheath according to any example herein, particularly examples 37-47, wherein the plurality of folded regions are heat set into the tip portion.

Example 49: The delivery sheath according to any example herein, particularly examples 37-48, wherein the plurality of folded regions are biased to the folded configuration.

Example 50: The delivery sheath according to any example herein, particularly examples 37-49, wherein the ring portion defines an elongated tubular structure having a central lumen extending therethrough.

Example 51: The delivery sheath according to any example herein, particularly examples 37-50, wherein the ring portion comprises a cylindrically-shaped outer surface.

Example 52: The delivery sheath according to any example herein, particularly examples 37-51, wherein the central lumen of the ring portion comprises a cylindrically-shaped inner surface.

Example 53: The delivery sheath according to any example herein, particularly examples 37-52, wherein an inner diameter of a central lumen extending through the ring portion corresponds to an inner diameter of a central lumen of the sheath member.

Example 54: The delivery sheath according to any example herein, particularly examples 37-53, wherein the ring portion and the sheath member form a continuous central lumen extending between the proximal end of the sheath member and a distal end of the ring portion.

Example 55: The delivery sheath according to any example herein, particularly examples 37-54, wherein a thickness of the ring portion corresponds to a thickness of the sheath member.

Example 56: The delivery sheath according to any example herein, particularly examples 37-54, wherein the thickness of the ring portion is greater than the thickness of the sheath member.

Example 57: The delivery sheath according to any example herein, particularly examples 37-54, wherein the ring portion has an elastic modulus corresponding to an elastic modulus of the sheath member.

Example 58: The delivery sheath according to any example herein, particularly examples 37-54, wherein the ring portion has an elastic modulus greater than an elastic modulus of the sheath member.

Example 59: The delivery sheath according to any example herein, particularly examples 37-54, wherein the ring portion has an elastic modulus less than an elastic modulus of the sheath member.

Example 60: The delivery sheath according to any example herein, particularly examples 37-59, wherein the ring portion has a uniform thickness along its length.

Example 61: The delivery sheath according to any example herein, particularly examples 37-60, wherein the ring portion has a uniform thickness around its circumference.

Example 62: The delivery sheath according to any example herein, particularly examples 37-61, wherein a length of the ring portion is less than a length of the tip portion.

Example 63: The delivery sheath according to any example herein, particularly examples 37-61, wherein a length of the ring portion corresponds to a length of the tip portion.

Example 64: The delivery sheath according to any example herein, particularly examples 37-61, wherein a length of the ring portion is greater than a length of the tip portion.

Example 65: The delivery sheath according to any example herein, particularly example 64, wherein the length of the ring portion is at least twice the length of the tip portion.

Example 66: The delivery sheath according to any example herein, particularly examples 37-65, wherein the ring portion is formed as a separate structure and coupled to the distal end of the sheath member.

Example 67: The delivery sheath according to any example herein, particularly examples 37-65, wherein the ring portion is integrally formed with the sheath member.

Example 68: The delivery sheath according to any example herein, particularly examples 37-67, wherein the tip portion defines a continuous tubular structure having a central lumen extending therethrough.

Example 69: The delivery sheath according to any example herein, particularly examples 37-68, wherein a diameter of the tip portion in the folded configuration corresponds to a diameter of the ring portion, wherein a diameter of the tip portion in the unfolded configuration is greater than a diameter of the ring portion.

Example 70: The delivery sheath according to any example herein, particularly examples 37-69, wherein the tip portion is formed from a conical tubular structure.

Example 71: The delivery sheath according to any example herein, particularly examples 37-70, wherein the tip portion further comprises a conically-shaped outer surface when the tip portion is in the unfolded configuration.

Example 72: The delivery sheath according to any example herein, particularly examples 37-71, wherein the tip portion further comprises a conically-shaped inner surface when the tip portion is in the unfolded configuration.

Example 73: The delivery sheath according to any example herein, particularly examples 37-72, wherein at least a portion of the tip portion further comprises a cylindrically-shaped inner and outer surface.

Example 74: The delivery sheath according to any example herein, particularly examples 37-73, wherein an inner diameter of a central lumen extending through the tip portion corresponds to an inner diameter of a central lumen of the ring portion.

Example 75: The delivery sheath according to any example herein, particularly examples 37-74, wherein the tip portion and the ring portion form a continuous central lumen extending between a proximal end of the ring portion and a distal end of the tip portion.

Example 76: The delivery sheath according to any example herein, particularly examples 37-75, wherein a thickness of the tip portion corresponds to a thickness of the ring portion.

Example 77: The delivery sheath according to any example herein, particularly examples 37-76, wherein the thickness of the tip portion is less than the thickness of the ring portion.

Example 78: The delivery sheath according to any example herein, particularly examples 37-77, wherein the thickness of the tip portion is less than the thickness of the sheath member.

Example 79: The delivery sheath according to any example herein, particularly examples 37-78, wherein the thickness of the tip portion is non-uniform along the length of the tip portion.

Example 80: The delivery sheath according to any example herein, particularly examples 37-79, wherein the tip portion has an elastic modulus less than an elastic modulus of the ring portion.

Example 81: The delivery sheath according to any example herein, particularly examples 37-79, wherein the tip portion has an elastic modulus corresponding to an elastic modulus of the ring portion.

Example 82: The delivery sheath according to any example herein, particularly examples 37-81, wherein the tip portion has a uniform thickness along its length.

Example 83: The delivery sheath according to any example herein, particularly examples 37-82, wherein the tip portion has a uniform thickness around its circumference.

Example 84: The delivery sheath according to any example herein, particularly examples 37-83, wherein a length of the tip portion corresponds to a length of the ring portion.

Example 85: The delivery sheath according to any example herein, particularly examples 37-83, wherein a length of the tip portion is less than a length of the ring portion.

Example 86: The delivery sheath according to any example herein, particularly examples 37-83, wherein a length of the tip portion is greater than a length of the ring portion.

Example 87: The delivery sheath according to any example herein, particularly examples 37-86, wherein the tip portion is formed as a separate structure and coupled to the distal end of the ring portion.

Example 88: The delivery sheath according to any example herein, particularly examples 37-86, wherein the tip portion is integrally formed with the ring portion.

Example 89: The delivery sheath according to any example herein, particularly examples 37-88, wherein the sheath member, the ring portion, and the tip portion are all constructed from a same material.

Example 90: The delivery sheath according to any example herein, particularly examples 37-88, wherein the sheath member, the ring portion, and the tip portion are all constructed from different materials.

Example 91: The delivery sheath according to any example herein, particularly examples 37-90, further comprising a self-expandable prosthetic heart valve, wherein central lumens of both the sheath member and the ring portion are sized and configured to receive the prosthetic heart valve in a compressed configuration, wherein the tip portion moves from the folded to the unfolded configuration in response to the outward directed radial force of the prosthetic heart valve as it passes through the tip portion, wherein movement from the folded configuration to the unfolded configuration causes the tip portion to transition from an elongated cylindrical shape to a conical shape defining an increasing tapered inner surface, where the diameter of the distal end of the tip portion is greater than a diameter of a proximal end of the tip portion.

Example 92: The delivery sheath according to any example herein, particularly example 91, wherein the prosthetic heart valve at least partially expands from a compressed configuration towards an expanded configuration as it passes through the tip portion.

Example 93: The delivery sheath according to any example herein, particularly examples 91 and 92, wherein the tip portion is biased towards the folded configuration such that the tip portion moves towards the folded configuration after the prosthetic heart valve passes through the tip portion.

Example 94: A method of deploying a prosthetic valve comprising: inserting an introducer sheath into a blood vessel; inserting a delivery sheath into a central lumen of the introducer sheath, with a prosthetic valve disposed therein, the delivery sheath comprising: a sheath member defining a tubular structure extending between a proximal end and a distal end of the sheath member; a ring portion provided at the distal end of the sheath member; a tip portion extending from a distal end of the ring portion, the tip portion including a longitudinally extending folding region movable between a folded and unfolded configuration, in the folded configuration the tip portion creases along a longitudinally extending edge such that circumferential portions of the tip portion at least partially overlap, in the unfolded configuration, the tip portions defines an increasing tapered surface having a larger diameter at a distal end of the tip portion; advancing a prosthetic valve through the distal end of the sheath member, to the ring portion; advancing the prosthetic valve through the ring portion to the tip portion; advancing the prosthetic valve through the tip portion such that the prosthetic valve at least partially expands within the tip portion and the tip portion moves from the folded to the unfolded configuration in response to the outward directed radial force of the prosthetic heart valve passing through the tip portion; advancing the prosthetic valve beyond a distal end of the delivery sheath to a treatment site.

Example 95: The method according to any example herein, particularly example 94, wherein the tip portion is biased towards a folded configuration such that the tip portion returns toward the folded configuration after the prosthetic heart valve has fully passed through the tip portion.

Example 96: The method according to any example herein, particularly examples 94 and 95, wherein the folding region is defined by a first (outer) circumferential portion, a first and second longitudinal edges, and a second (intermediate) circumferential portion extending between the first and second longitudinal edges, such that the first folding region is configured to crease at the first and second longitudinal edges into the folded configuration such that the first and second circumferential portions at least partially overlap, and the second circumferential portion is radially inward of the first circumferential portion, wherein advancing the prosthetic valve through the tip portion causes the first and second circumferential portions to move to a less overlapping position than when in the folded configuration, wherein advancing the prosthetic valve through the tip portion causes the first and second circumferential portions to circumferentially align when the tip portion is a fully unfolded configuration.

Example 97: The method according to any example herein, particularly examples 94 and 95, further comprising withdrawing the prosthetic valve from the treatment site by moving the prosthetic valve in a direction towards the sheath member such that the prosthetic valve is withdrawn (e.g., fully or partially) into the tip portion, wherein the prosthetic valve is disposed in an at least a partially expanded configuration when outside of the delivery sheath, and wherein a tapered inner surface of the tip portion facilitates the compression (e.g., folding and/or crimping) of the prosthetic heart valve as it is withdrawn into the tip portion and towards the sheath member.

Example 98: The method according to any example herein, particularly of example 97, wherein withdrawing the prosthetic valve from the treatment site further comprises: retaining the prosthetic valve in the sheath member and/or the ring portion, removing the delivery sheath from the blood vessel, and removing the delivery sheath from the introducer sheath.

Example 99: The method according to any example herein, particularly of examples 94-98, wherein the prosthetic valve is a self-expanding heart valve.

Example 100: The method according to any example herein, particularly of examples 94-99, wherein the prosthetic valve is an Aortic valve.

Example 101: A delivery sheath comprising: a sheath member defining a tubular structure extending between a proximal end and a distal end of the sheath member; a tip portion provided at the distal end of the sheath member, the tip portion having a central lumen extending therethrough, wherein the tip portion is discontinuous having two flaps extending circumferentially around the tip portion, wherein the tip portion is movable between an unexpanded and an expanded configuration, in the unexpanded configuration the tip portion defines a constant diameter along its length, in the expanded configuration, the tip portion flares open increasing a diameter of the distal end of the tip portion and increasing the spacing between the two flaps.

Example 102: The delivery sheath according to any example herein, particularly of example 101, wherein the two flaps include a first flap and a second flap formed symmetrically about the tip portion such that a circumferential width of the first flap corresponds to a circumferential width of the second flap.

Example 103: The delivery sheath according to any example herein, particularly of examples 101 and 102, wherein the two flaps include a first flap and a second flap formed asymmetrically about the tip portion such that a circumferential width of the first flap is greater than a circumferential width of the second flap.

Example 104: The delivery sheath according to any example herein, particularly of examples 101-103, further comprising a third flap provided between the first and the second flap, wherein the first, second and third flaps are formed symmetrically about a circumference of the tip portion.

Example 105: The delivery sheath according to any example herein, particularly of examples 101-104, wherein, in the unexpanded configuration, the first, second and third flaps are formed around the tip portion with a gap provided between adjacent flaps, wherein each of the first, second and third flaps include a leading side edge and a trailing side edge, wherein in the unexpanded configuration the gap is provided between adjacent leading and trailing side edges of each of the first, second and third flaps.

Example 106: The delivery sheath according to any example herein, particularly of examples 101-104, wherein, in the unexpanded configuration, the first, second and third flaps are coupled to each other along weakened side edges, wherein movement of the tip portion from the unexpanded to the expanded configuration causes the first, second and third flaps to separate along the weakened side edges to increase the spacing between adjacent flaps.

Example 107: The delivery sheath according to any example herein, particularly of example 106, wherein the weakened side edge is formed from a perforation, a thinned portion, a crease, or a combination thereof.

Example 108: The delivery sheath according to any example herein, particularly of examples 101-107, further comprising: a ring portion provided between the distal end of the sheath member and a proximal end of the tip portion.

Example 109: The delivery sheath according to any example herein, particularly of example 108, wherein the ring portion defines an elongated tubular structure having a central lumen extending therethrough, wherein the tip portion, the ring portion and the sheath member form a continuous central lumen extending between the proximal end of the sheath member and a distal end of the tip portion.

Example 110: The delivery sheath according to any example herein, particularly of examples 108 and 109, wherein an inner diameter of a central lumen extending through the ring portion corresponds to an inner diameter of a central lumen of the sheath member.

Example 111: The delivery sheath according to any example herein, particularly of examples 108-110, wherein a thickness of the ring portion corresponds to a thickness of the sheath member.

Example 112: The delivery sheath according to any example herein, particularly of examples 108-110, wherein the thickness of the ring portion is greater than the thickness of the sheath member.

Example 113: The delivery sheath according to any example herein, particularly of examples 108-112, wherein the ring portion has an elastic modulus less than an elastic modulus of the sheath member.

Example 114: The delivery sheath according to any example herein, particularly of examples 108-112, wherein the ring portion has an elastic modulus corresponding to an elastic modulus of the sheath member.

Example 115: The delivery sheath according to any example herein, particularly of examples 108-114, wherein the ring portion has a uniform thickness along its length.

Example 116: The delivery sheath according to any example herein, particularly of examples 108-115, wherein the ring portion has a uniform thickness around its circumference.

Example 117: The delivery sheath according to any example herein, particularly of examples 108-116, wherein a length of the ring portion is less than a length of the tip portion.

Example 118: The delivery sheath according to any example herein, particularly of examples 108-116, wherein a length of the ring portion corresponds to a length of the tip portion.

Example 119: The delivery sheath according to any example herein, particularly of examples 108-116, wherein a length of the ring portion is greater than a length of the tip portion.

Example 120: The delivery sheath according to any example herein, particularly of example 119, wherein the length of the ring portion is at least twice the length of the tip portion.

Example 121: The delivery sheath according to any example herein, particularly of examples 108-120, wherein the ring portion is formed as a separate structure and coupled to the distal end of the sheath member.

Example 122: The delivery sheath according to any example herein, particularly of examples 108-120, wherein the ring portion is integrally formed with the sheath member.

Example 123: The delivery sheath according to any example herein, particularly of examples 108-122, wherein the sheath member and the ring portion are constructed from a same material.

Example 124: The delivery sheath according to any example herein, particularly of examples 101-123, wherein a proximal end of the tip portion is coupled to the distal end of the sheath member, wherein a diameter of the proximal end of the tip portion corresponds to a diameter of a distal end of the sheath portion.

Example 125: The delivery sheath according to any example herein, particularly of examples 101-124, wherein a diameter of the tip portion in the unexpanded configuration corresponds to a diameter of at least one of the sheath member and the ring portion, wherein a diameter of the tip portion in the expanded configuration is greater than a diameter of at least one of the sheath member and the ring portion.

Example 126: The delivery sheath according to any example herein, particularly of examples 101-125, wherein an inner surface of the tip portion at its proximal end defines a circular-shape in cross-section when the tip portion is in an unexpanded configuration.

Example 127: The delivery sheath according to any example herein, particularly of examples 101-126, wherein the inner surface of the tip portion forms an elongated cylindrical shape when in the unexpanded configuration.

Example 128: The delivery sheath according to any example herein, particularly of examples 101-127, wherein the tip portion, the ring portion and the sheath member form a continuous central lumen extending between a proximal end of the sheath member and a distal end of the tip portion.

Example 129: The delivery sheath according to any example herein, particularly of examples 101-128, wherein a thickness of the tip portion corresponds to a thickness of at least one of the ring portion and the sheath member.

Example 130: The delivery sheath according to any example herein, particularly of examples 101-129, wherein the thickness of the tip portion is less than the thickness of at least one of the ring portion and the sheath member.

Example 131: The delivery sheath according to any example herein, particularly of examples 101-130, wherein the tip portion has an elastic modulus less than an elastic modulus of the ring portion and an elastic modulus of the sheath member.

Example 132: The delivery sheath according to any example herein, particularly of examples 101-131, wherein the tip portion is formed from a superelastic material.

Example 133: The delivery sheath according to any example herein, particularly of examples 101-132, wherein the tip portion is formed from a metal alloy.

Example 134: The delivery sheath according to any example herein, particularly of example 133, wherein the metal alloy is covered with a plastic.

Example 135: The delivery sheath according to any example herein, particularly of examples 101-134, wherein the tip portion has a uniform thickness along its length.

Example 136: The delivery sheath according to any example herein, particularly of examples 101-135, wherein the tip portion has a uniform thickness around its circumference.

Example 137: The delivery sheath according to any example herein, particularly of examples 101-136, wherein a length of the tip portion corresponds to a length of the ring portion.

Example 138: The delivery sheath according to any example herein, particularly of examples 101-136, wherein a length of the tip portion is less than a length of the ring portion.

Example 139: The delivery sheath according to any example herein, particularly of examples 101-136, wherein a length of the tip portion is greater than a length of the ring portion.

Example 140: The delivery sheath according to any example herein, particularly of examples 101-139, wherein the tip portion is formed as a separate structure and coupled to the distal end of the ring portion.

Example 141: The delivery sheath according to any example herein, particularly of examples 101-139, wherein the tip portion is integrally formed with the ring portion.

Example 142: The delivery sheath according to any example herein, particularly of examples 101-141, further comprising a self-expandable prosthetic heart valve, wherein central lumens of both the sheath member and the ring portion are sized and configured to receive the prosthetic heart valve in a compressed configuration, wherein the tip portion moves from the unexpanded to the expanded configuration in response to the outward directed radial force of the prosthetic heart valve as it passes through the tip portion, wherein movement from the unexpanded configuration to the expanded configuration cause the flaps to flare open such that the diameter of the distal end of the tip portion is greater than a diameter of a proximal end of the tip portion.

Example 143: The delivery sheath according to any example herein, particularly of example 142, wherein the prosthetic heart valve at least partially expands from a compressed configuration towards an expanded configuration as it passes through the tip portion.

Example 144: The delivery sheath according to any example herein, particularly of examples 142-143, wherein the tip portion is biased towards the unexpanded configuration such that the tip moves towards the unexpanded configuration after the prosthetic heart valve passes through the tip portion.

Example 145: A method of deploying a prosthetic valve comprising: inserting an introducer sheath into a blood vessel; inserting a delivery sheath into a central lumen of the introducer sheath, with a prosthetic valve disposed therein, the delivery sheath comprising: a sheath member defining a tubular structure extending between a proximal end and a distal end of the sheath member; a tip portion provided at the distal end of the sheath member, the tip portion having a central lumen extending therethrough, where the tip portion is discontinuous having two flaps extending circumferentially around the tip portion, the tip portion is movable between an expanded and an unexpanded configuration, in the unexpanded configuration the tip portion defines a constant diameter along its length, in the expanded configuration, the tip portion flares open increasing a diameter of the distal end of the tip portion and increasing the spacing between the two flaps; advancing a prosthetic valve through the distal end of the sheath member, to the tip portion; advancing the prosthetic valve through the tip portion to a treatment site such that the prosthetic valve at least partially expands within the tip portion and the tip portion moved from the unexpanded to the expanded configuration in response to the outward directed radial force of the prosthetic heart valve passing through the tip portion; advancing the prosthetic valve beyond a distal end of the delivery sheath to a treatment site.

Example 146: The method according to any example herein, particularly of example 145, wherein the tip portion is biased towards an unexpanded configuration such that the tip portion returns toward the unexpanded configuration after the prosthetic heart valve has fully passed through the tip portion.

Example 147: The method according to any example herein, particularly of examples 145 and 146, wherein, in the unexpanded configuration, the first and second flaps are formed around the tip portion with longitudinally extending gaps provided between the longitudinal sides of each of the first and second flap, wherein advancing the prosthetic valve through the tip portion causes the gaps between the longitudinal sides of each of the first and second flap to increase.

Example 148: The method according to any example herein, particularly of examples 145 and 146, wherein, in the unexpanded configuration, the first and second flaps are coupled to each other along weakened side edges, wherein advancing the prosthetic valve through the tip portion causes the first and second flaps to separate along the weakened side edges to increase the spacing between adjacent flaps.

Example 149: The method according to any example herein, particularly of examples 145-148, further comprising withdrawing the prosthetic valve from the treatment site by moving the prosthetic valve in a direction towards the sheath member such that the prosthetic valve is withdrawn (e.g., fully or partially) into the tip portion, wherein the prosthetic valve is disposed in an at least a partially expanded configuration when outside of the delivery sheath, and wherein a tapered inner surface of the tip portion facilitates the compression (e.g., folding and/or crimping) of the prosthetic heart valve as it is withdrawn into the tip portion and towards the sheath member.

Example 150: The method according to any example herein, particularly of example 149, wherein withdrawing the prosthetic valve from the treatment site further comprises: retaining the prosthetic valve in the sheath member and/or the ring portion, removing the delivery sheath from the blood vessel, and removing the delivery sheath from the introducer sheath.

Example 151: The method according to any example herein, particularly of examples 145-150, wherein the prosthetic valve is a self-expanding heart valve.

Example 152: The method according to any example here, particularly of examples 145-151, wherein the prosthetic valve is an Aortic valve.

The invention further comprises the following embodiments:

1. A delivery sheath system comprising: a sheath member defining a tubular structure extending between a proximal end and a distal end of the sheath member; a tip portion provided at the distal end of the sheath member, the tip portion having a central lumen extending therethrough, wherein at least one cross-sectional segment of an inner surface of the tip portion includes alternating portions of concave and convex curvature.

2. The delivery sheath system of embodiment 1, wherein the alternating portions of concave and convex curvature are distributed evenly about a circumference of the tip portion.

3. The delivery catheter of any one of embodiments 1-2, wherein a radius of curvature of the portion of concave curvature is equal to a radius of curvature of the portion of convex curvature.

4. The delivery catheter of any one of embodiments 1-2, wherein a radius of curvature of the portion of concave curvature is greater than a radius curvature of the portion of convex curvature.

5. The delivery catheter of any one of embodiments 1-2, wherein a radius of curvature of the portion of concave curvature is less than a radius of curvature of the portion of convex curvature.

6. The delivery sheath system of any one of embodiments 1-5, including at least two portions of concave curvature and at least two portions of convex curvature.

7. The delivery sheath system of any one of embodiments 1-6, wherein a proximal end of the tip portion is coupled to the distal end of the sheath member, wherein a diameter of the proximal end of the tip portion corresponds to a diameter of a distal end of the sheath portion.

8. The delivery sheath system of any one of embodiments 1-7, wherein the portions of concave and convex curvature extend longitudinally along the tip portion.

9. The delivery sheath system of any one of embodiments 1-8, wherein the portions of concave and convex curvature extend longitudinally along the tip portion and define a decreasing tapered surface extending in a longitudinal direction between the distal and proximal ends of the tip portion.

10. The delivery sheath system of any one of embodiments 1-9, wherein an apex of at least one of the portions of convex curvature (e.g., adjacent the proximal end of the tip portion) defines a minimum radius of the inner surface of the tip portion.

11. The delivery sheath system of any one of embodiments 1-10, wherein an apex of at least one of the portions of concave curvature (e.g., adjacent the distal end of the tip portion) defines a maximum radius of the inner surface of the tip portion.

12. The delivery sheath system of any one of embodiments 1-11, wherein the tip portion further comprises an outer surface, wherein at least one cross-sectional segment of the outer surface includes alternating portions of concave and convex curvature, and wherein the alternating portions of concave and convex curvature of the outer surface are aligned (e.g., circumferentially and/or radially aligned) with the alternating portions of concave and convex curvature of the inner surface.

13. The delivery sheath system of any one of embodiments 1-12, wherein the tip portion further comprises a cylindrically-shaped outer surface.

14. The delivery sheath system of any one of embodiments 1-13, further comprising a nose cone, the nose cone having a proximal end and a tapering distal end and an intermediate section therebetween, wherein the proximal end of the nose cone is detachably coupled to the distal end of the tip portion, wherein at least a portion of an outer surface of the intermediate section of the nose cone includes alternating portions of concave and convex curvature corresponding to the inner surface of the tip portion.

15. A method of deploying a prosthetic valve through an introducer sheath comprising: inserting a delivery sheath into a central lumen of the introducer sheath, with a prosthetic valve disposed therein, the delivery sheath comprising: a sheath member defining a tubular structure extending between a proximal end and a distal end of the sheath member; a tip portion provided at the distal end of the sheath member, the tip portion having a central lumen extending therethrough, wherein at least one cross-sectional segment of an inner surface of the tip portion includes alternating portions of concave and convex curvature; advancing a prosthetic valve through the distal end of the sheath member, to the tip portion; advancing the prosthetic valve through the tip portion such that the prosthetic valve at least partially expands within the tip portion, wherein the alternating portions of concave and convex curvature facilitate directed expansion of the prosthetic heart valve as it passes through the tip portion, wherein sections of the prosthetic valve are compressed at circumferential locations corresponding to the portions of convex curvature (e.g., compressed greater at the circumferential locations corresponding to the portions of convex curvature than compared to compression of the valve at those portions of concave curvature).

16. The method of embodiment 15, wherein the delivery sheath further comprises, a nose cone, having a proximal end and a tapering distal end, the nose cone detachably coupled to the distal end of the tip portion;

the method further comprising advancing the nose cone away from the distal end of the tip portion.

17. A delivery sheath system comprising: a sheath member defining a tubular structure extending between a proximal end and a distal end of the sheath member; a ring portion provided at the distal end of the sheath member; and a tip portion extending from a distal end of the ring portion, the tip portion including a longitudinally extending folding region movable between a folded and unfolded configuration, in the folded configuration the tip portion creases along a longitudinally extending edge such that circumferential portions of the tip portion at least partially overlap, wherein, in the unfolded configuration, the tip portion defines an increasing tapered surface having a larger diameter at a distal end of the tip portion.

18. The delivery sheath of embodiment 17, wherein the tip portion includes a plurality of folding regions circumferentially spaced around the tip portion.

19. The delivery sheath of embodiment 17-18, wherein the plurality of folding regions include a first folding region defined by a first (outer) circumferential portion, a first and second longitudinal edges, and a second (intermediate) circumferential portion extending between the first and second longitudinal edges, wherein the first folding region is configured to crease at the first and second longitudinal edges into the folded configuration such that the first and second circumferential portions at least partially overlap, and the second circumferential portion is radially inward of the first circumferential portion.

20. The delivery sheath of any one of embodiment 17-19, wherein the plurality of folding regions include a second folding region defined by a third (outer) circumferential portion, a third and fourth longitudinal edge, and a fourth (intermediate) circumferential portion extending between the third and fourth longitudinal edges, wherein the second folding region is configured to crease at the third and fourth longitudinal edges into the folded configuration such that the third and fourth circumferential portions at least partially overlap, and the fourth circumferential portion is radially inward of the third circumferential portion.

21. The delivery sheath of embodiments 17-20, wherein the first folding region and the second folding region are spaced circumferentially around the tip portion on opposing sides of a fifth circumferential region, wherein the fifth circumferential region extends between the second and third longitudinal edges.

## Claims

1. A delivery sheath system for percutaneous delivery and implantation of a prosthetic heart valve, the delivery sheath system comprising:

a sheath member (106) defining a tubular structure extending between a proximal end and a distal end of the sheath member (106);
a ring portion (302) provided at the distal end of the sheath member (106); and
a tip portion (300) extending from a distal end of the ring portion, the tip portion (300) including a longitudinally extending folding region (304) movable between a folded and unfolded configuration, in the folded configuration the tip portion (300) creases along a longitudinally extending edge (314, 316) such that circumferential portions (312, 330) of the tip portion (300) at least partially overlap,
wherein, in the unfolded configuration, the tip portion (300) defines an increasing tapered surface having a larger diameter at a distal end of the tip portion (300); and
wherein the tip portion is biased towards the folded configuration such that the tip portion (300) moves towards the folded configuration after a prosthetic heart valve passes through the tip portion (300).

2. The delivery sheath system of claim 1, wherein the tip portion (300) includes a plurality of folding regions circumferentially spaced around the tip portion (300).

3. The delivery sheath system of claim 2, wherein the plurality of folding regions include a first folding region (310) defined by a first circumferential portion (312), a first and second longitudinal edges (314, 316), and a second circumferential portion (318) extending between the first and second longitudinal edges (314, 316),
wherein the first folding region (310) is configured to crease at the first and second longitudinal edges (314, 316) into the folded configuration such that the first and second circumferential portions (312, 318) at least partially overlap, and the second circumferential portion (318) is radially inward of the first circumferential portion (312).

4. The delivery sheath system of claim 3, wherein the plurality of folding regions include a second folding region (320) defined by a third circumferential portion (322), a third and fourth longitudinal edge (324, 326), and a fourth circumferential portion (328) extending between the third and fourth longitudinal edges (324, 326),
wherein the second folding region (320) is configured to crease at the third and fourth longitudinal edges (324, 326) into the folded configuration such that the third and fourth circumferential portions (322, 328) at least partially overlap, and the fourth circumferential portion (328) is radially inward of the third circumferential portion (322).

5. The delivery sheath system of claim 4, wherein the first folding region (310) and the second folding region (320) are spaced circumferentially around the tip portion (300) on opposing sides of a fifth circumferential region (330).

6. The delivery sheath system of claim 5, wherein the fifth circumferential region (330) extends between the second and third longitudinal edges (316, 324).

7. The delivery sheath system of any one of claims 5 or 6, wherein, in the folded configuration, the fifth circumferential portion (330) is radially inward of the second and fourth circumferential portions (322, 328).

8. The delivery sheath system of any one of claims 5 to 7, wherein a width of the fifth circumferential portion (330) measured around the circumference of the tip portion (300) is less than a width of the first and third circumferential portions (312, 328).

9. The delivery sheath system of any one of claims 5 to 8, wherein a width of either of the second and fourth circumferential portions (322, 328) is less than the width of the fifth circumferential portion (330).

10. The delivery sheath system of any one of claims 2 to 9, wherein the plurality of folding region are asymmetrically spaced around a circumference of the tip portion (300).

11. The delivery sheath system of any one of claims 2 to 9, wherein the plurality of folding region are symmetrically spaced around a circumference of the tip portion (300).

12. The delivery sheath system of any one of claims 2 to 11, wherein the plurality of folded regions are heat set into the tip portion (300).

13. The delivery sheath system of any one of claims 2 to 12, wherein the plurality of folded regions are biased to the folded configuration.

14. The delivery sheath system of any one of claims 1 to 13, wherein the ring portion (302) has an elastic modulus greater than an elastic modulus of the sheath member (106).

15. The delivery sheath system of any one of claims 1 to 14, wherein the ring portion (302) is integrally formed with the sheath member (106).

FIG. 1

FIG. 2

FIG. 3

FIG. 4

FIG. 5A

FIG. 5B

FIG. 6

FIG. 7

FIG. 8

FIG. 9

FIG. 10

FIG. 11A

FIG. 11B

FIG. 12

FIG. 13

FIG. 14

FIG. 15

FIG. 16

FIG. 17

FIG. 18

FIG. 19

FIG. 20

FIG. 21

EP 4 559 504 A2

FIG. 22

FIG. 23

FIG. 24

FIG. 25

200

10

106

FIG. 26

FIG. 27

106

302

300

304

FIG. 28

106

302

300

FIG. 29

FIG. 30

FIG.31

FIG.32

## REFERENCES CITED IN THE DESCRIPTION

**Patent documents cited in the description**

- US 63005905
- US 63076783
- US 63110846
- US 9867700 B
- US 6730118 B
- US 20080065011 A
- US 8652202 B
- US 9155619 B
- US 20100049313
- US 429040